# EUROPEAN PATENT APPLICATION

(11) **EP 2 402 727 A1**
(43) Date of publication of application: **04.01.2012**
(21) Application number: 09840849.5
(22) Date of filing: 15.12.2009
(51) Int. Cl.: G01J 5/00, C07D 487/22, C09B 47/04, C09B 47/18, C09D 11/02, C09K 3/00, G01J 5/48, B42D 15/10

(54) **METHOD FOR PROVING AUTHENTICITY USING NAPHTHALOCYANINE COMPOUND, SIGNAL TRANSDUCTION METHOD, POLYMER WELDING METHOD, METHOD FOR PRODUCING LITHOGRAPHIC PRINTING PLATE, PRINTING INK, TONER, HEAT-SHIELDING MATERIAL, AND METHOD FOR PRODUCING NAPHTHALOCYANINE COMPOUND**

(30) Priority: 26.02.2009 JP 2009044510
(71) Applicant: FUJIFILM Corporation, Tokyo 106-8620 (JP)
(72) Inventor: KIMURA, Keizo, Odawara-shi Kanagawa 250-0001 (JP); AMEMIYA, Takuma, Odawara-shi Kanagawa 250-0001 (JP); KOBAYASHI, Katsumi, Odawara-shi Kanagawa 250-0001 (JP)
(74) Representative: HOFFMANN EITLE
(86) International application number: PCT/JP2009/070905
(87) International publication number: WO 2010/098002

(57) **Abstract**

Disclosed is a method of proving the authenticity of goods or a support comprising using a compound represented by Formula (I). 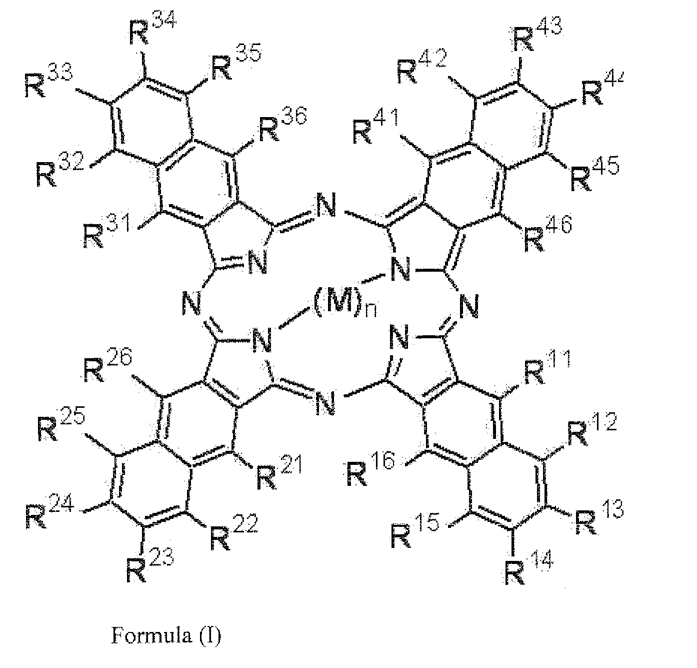 wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded each other to form a ring; M represents a hydrogen atom, a metal ion, or a group containing a metal ion; and n represents 1. or 2. The infrared absorption efficiency is high and the deterioration in infrared absorption over time is ameliorated. The infrared absorption efficiency is high and the deterioration in infrared absorption over time is ameliorated.

## Description

### Technical Field

The present invention relates to a method of proving authenticity of goods, a signal conversion method, a polymer welding method, a method of producing a lithographic printing plate, an ink for printing, a toner, and a heat ray-shielding material each using a naphthalocyanine compound, specifically a specific naphthalocyanine compound that absorbs the electromagnetic spectrum of a near infrared region of from 750 nm to 1500 nm, and in particular from 800 nm to 1000 nm and a method of producing a naphthalocyanine compound that is suitable for such use.

### Background Art

Conventionally, various systems using a pigment that absorbs near infrared rays have been known. For example, in Japanese Translation of PCT International Application (JP-T) Nos. 2005-537,319 and 2001-527,600, a system using invisible information, a system for converting light energy to heat energy, and the like are disclosed. In Japanese Patent Application Laid-Open (JP-A) No. 2000-17,184, a system for shielding a heat ray is disclosed.

Different physical properties are required for each system with such uses. That is, in addition to a case in which having invisibility, namely, not having any absorption in the visible range, is the most important, it is also required to have an absorption pattern suitable for a wavelength of a laser or light-emitting source, or the like. Further, for any use, it is required to have at least a certain level of pigment fastness.

Further, as described in "Der Deutschen Chemischen Gesellschaft" vol. 92, pp. 245 to 251 (1959) and "Spectrochemica Acta" vol. 23, pp. 655 to 675 (1967), a tris-N,N,N-(4-aminophenyl)amine compound has been already known as an infrared-absorbing compound. However, study regarding its actual use as a near infrared absorbing pigment has not been carried out for the most part.

Methods of synthesizing a naphthalocyanine compound are known from JP-TNo. 8-508,269, Journal of Chemical Society Perkin Transaction, 1, page 2453 (1988), and Chemistry A European Journal, page 5123 (2003). However, for industrial use of the compound, it is required to further improve the yield in terms of productivity and cost.

### SUMMARY OF INVENTION

### Technical Problem

As discussed above, although various studies have been made regarding the use of a pigment that absorbs near infrared rays, a certain level or higher of pigment fastness is not necessarily satisfied in any use, and therefore, further improvement has been needed. In particular, excellent light resistance, heat resistance, and wet heat resistance are important.

The invention has been made in view of the above, and has an object of providing a method of proving authenticity of goods or a support having high infrared absorption efficiency (preferably the electromagnetic spectrum of near infrared rays of from 750 nm to 1500 nm, and in particular from 800 nm to 1000 nm) and ameliorated deterioration in infrared absorption over time, a signal conversion method, a polymer welding method, a method of producing a lithographic printing plate, an ink for printing, a toner, and a heat ray-shielding material, and a method of producing a naphthalocyanine compound, and relates to achieving this object.

### Solution to Problem

Specific means for achieving the object described above is as follows.
<1> A method of proving authenticity of goods or a support, comprising using a compound represented by the following Formula (I):

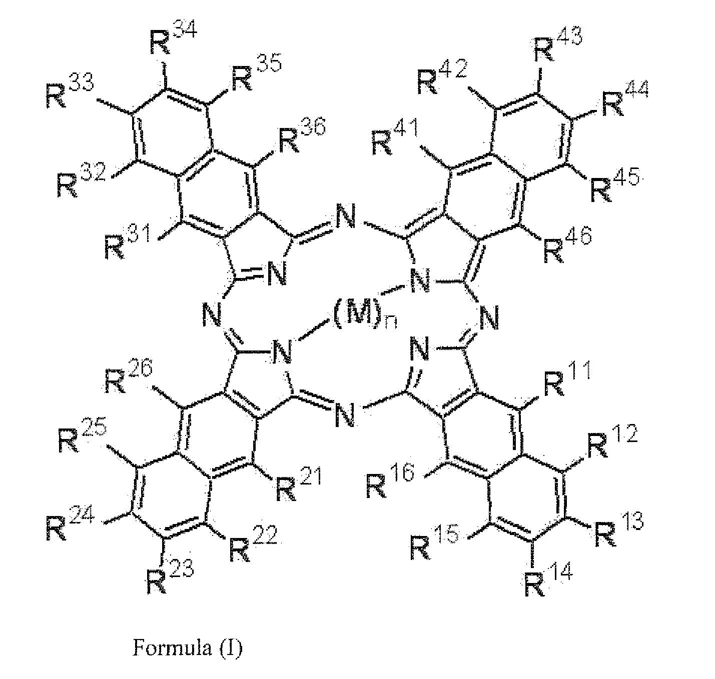

wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring; M represents a hydrogen atom, a metal ion, or a group containing a metal ion; and n represents 1 or 2.

<2> The method of proving authenticity according to <1>, wherein, in Formula (I), at least one of R¹¹, R¹⁶, R²¹, R²⁶, R³¹, R³⁶, R⁴¹, and R⁴⁶ represents an alkoxy group, an aryloxy group, an arylthio group, or a halogen atom.
<3> The method of proving authenticity according to <1> or <2>, wherein, in Formula (I), at least one of R¹¹, R¹⁶, R²¹, R²⁶, R³¹, R³⁶, R⁴¹, and R⁴⁶ represents an alkoxy group.
<4> The method of proving authenticity according to any one of <1> to <3>, comprising attaching, to a surface of the goods (or the support), an image using the compound represented by Formula (I), and detecting the image attached to the surface of the goods by irradiating with an infrared ray and scanning.
<5> The method of proving authenticity according to any one of <1> to <3>, comprising attaching, to the goods (or the support), a marking formed using the compound represented by Formula (I) and detecting or measuring a specific absorption by irradiating the marking with an infrared ray.
<6> The method of proving authenticity according to <5>, wherein the goods are mineral oil.
<7> A signal conversion method comprising converting a thermal signal into an enhanced thermal signal or converting a light signal into a thermal signal, by using a compound represented by the following Formula (I):

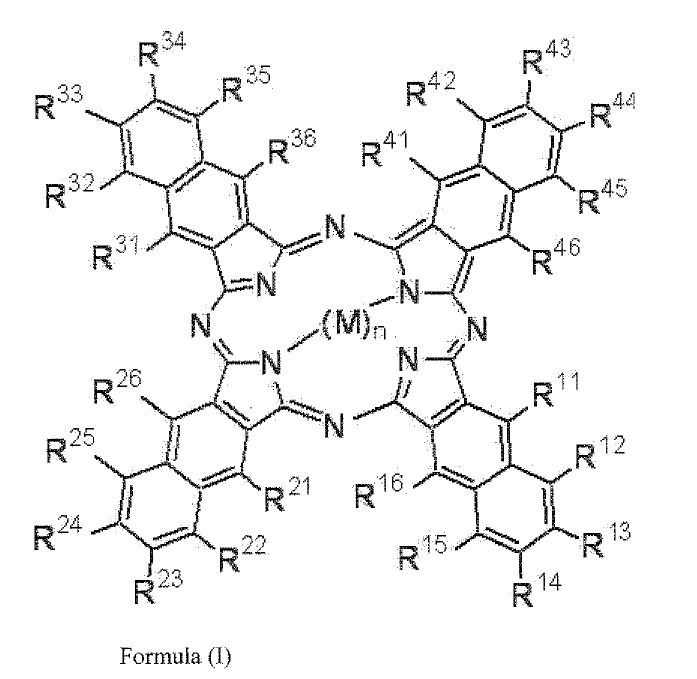

wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring; M represents a hydrogen atom, a metal ion, or a group containing a metal ion; and n represents 1 or 2.

<8> The signal conversion method according to <7>, wherein, in Formula (I), at least one of R¹¹, R¹⁶, R²¹, R²⁶, R³¹, R³⁶, R⁴¹, and R⁴⁶ represents an alkoxy group, an aryloxy group, an arylthio group, or a halogen atom.
<9> The signal conversion method according to <7> or <8>, wherein, in Formula (I), at least one of R¹¹, R¹⁶, R²¹, R²⁶, R³¹, R³⁶, R⁴¹, and R⁴⁶ represents an alkoxy group.
<10> A method of welding a polymer using the signal conversion method according to any one of <7> to <9>.
<11> A method of producing a lithographic printing plate using the signal conversion method according to any one of <7> to <9>, comprising irradiating a heat sensitive layer or photosensitive layer containing the compound represented by Formula (I) with an infrared laser in accordance with pattern information.
<12> An ink for printing, comprising a compound represented by the following Formula (I):

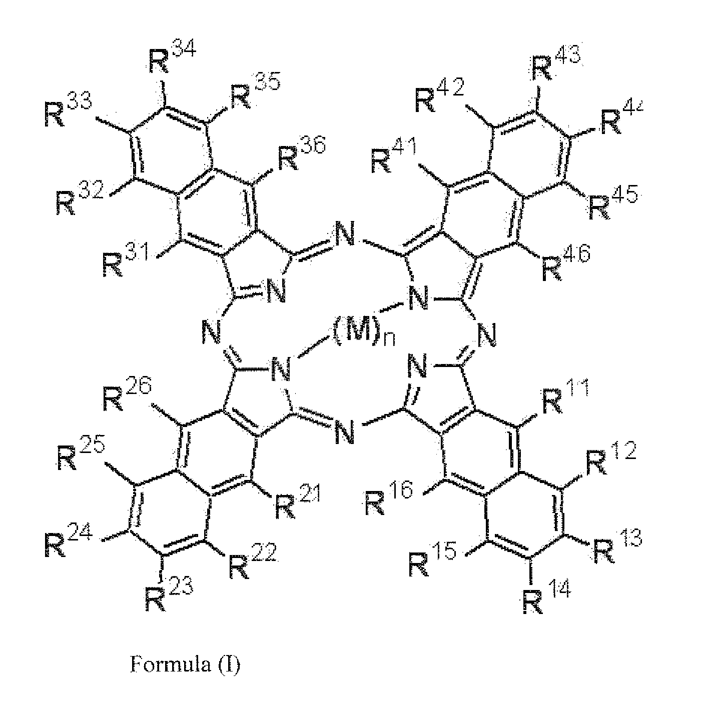

wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring; M represents a hydrogen atom, a metal ion, or a group containing a metal ion; and n represents 1 or 2.

<13> The ink for printing according to <12>, wherein, in Formula (I), at least one of R¹¹, R¹⁶, R²¹, R²⁶, R³¹, R³⁶, R⁴¹, and R⁴⁶ represents an alkoxy group, an aryloxy group, an arylthio group, or a halogen atom.
<14> The ink for printing according to <12> or <13>, wherein, in Formula (I), at least one of R¹¹, R¹⁶, R²¹, R²⁶, R³¹, R³⁶, R⁴¹, and R⁴⁶ represents an alkoxy group.
<15> The ink for printing according to any one of <12> to <14>, further comprising a colorant.
<16> The ink for printing according to any one of <12> to <15>, further comprising an alkoxylated or polyalkoxylated acrylate monomer and a photoinitiator.
<17> A toner comprising a compound represented by the following Formula (I):

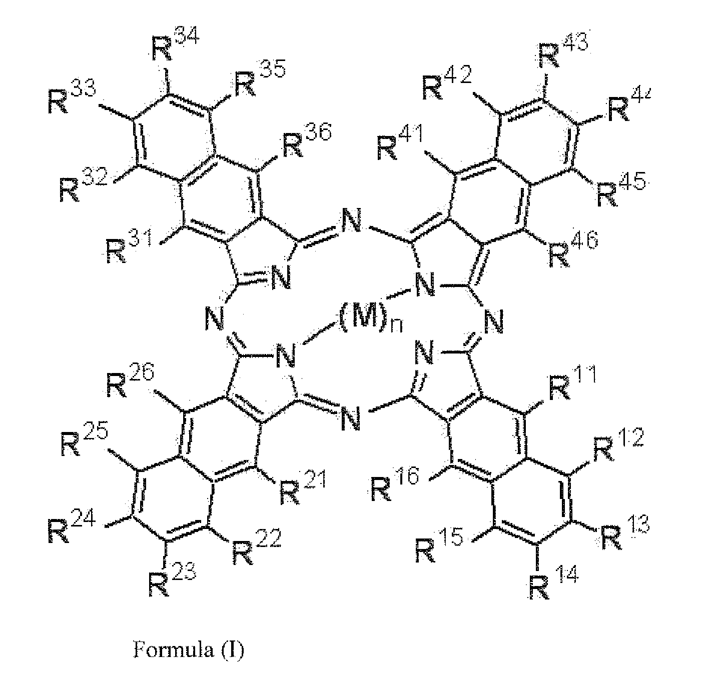

wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring; M represents a hydrogen atom, a metal ion, or a group containing a metal ion; and n represents I or 2.

<18> The toner according to <17>, wherein, in Formula (I), at least one of R¹¹, R¹⁶, R²¹, R²⁶, R³¹, R³⁶ R⁴¹, and R⁴⁶ represents an alkoxy group, an aryloxy group, an arylthio group, or a halogen atom.
<19> The toner according to <17> or <1 8>, wherein, in Formula (I), at least one of R¹¹, R¹⁶ R²¹, R²⁶, R³¹, R³⁶, R⁴¹, and R⁴⁶ represents an alkoxy group.
<20> The toner according to any one of <17> to <19>, further comprising a colorant.
<21 > The toner according to any one of <17> to <20>, further comprising an alkoxylated or polyalkoxylated acrylate monomer and a photoinitiator.
<22> A heat ray-shielding material comprising a compound represented by the following Formula (I):

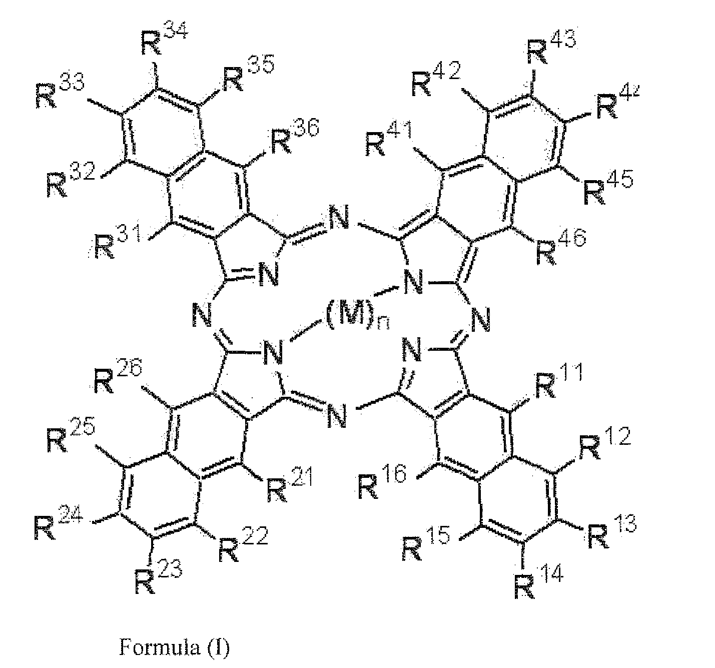

wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring; M represents a hydrogen atom, a metal ion, or a group containing a metal ion; and n represents 1 or 2.

<23> The heat ray-shielding material according to <22>, wherein, in Formula (I), at least one of R¹¹, R¹⁶, R²¹, R²⁶, R³¹, R³⁶, R⁴¹, and R⁴⁶ represents an alkoxy group, an aryloxy group, an arylthio group, or a halogen atom.
<24> The heat ray-shielding material according to <22> or <23>, wherein, in Formula (I), at least one of R¹¹, R¹⁶ , R²¹, R²⁶, R³¹, R³⁶, R⁴¹, and R⁴⁶ represents an alkoxy group.
<25> A method of producing a compound represented by Formula (II) comprising using t-BuOM³ and lithium halide in a solvent represented by one of Formulae R¹OH to R⁸OH or a mixture solvent of two or more thereof:

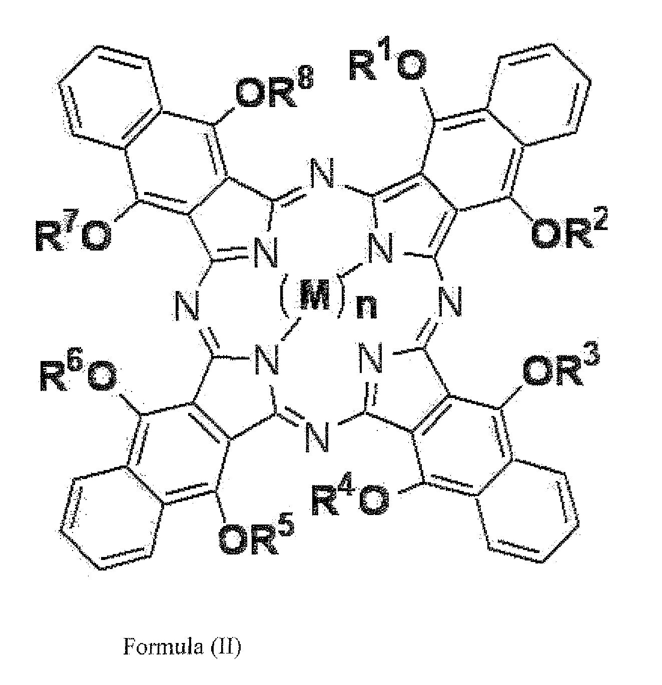

wherein, substituent groups R¹ to R⁸ each independently represent an alkyl group or an aryl group; M³ represents an alkali metal atom; M represents a hydrogen atom, a metal ion, or a group containing a metal ion; and n represents 1 or 2.

<26> The method of producing a compound represented by Formula (II) according to <25> comprising using hydroquinone.

### Advantageous Effects of Invention

According to the invention, a method of proving authenticity of goods or a support having high infrared absorption efficiency (preferably the electromagnetic spectrum of near infrared rays of from 750 nm to 950 nm, and in particular from 800 nm to 900 nm) and suppressed deterioration in infrared absorption over time, a signal conversion method, a polymer welding method, a method of producing a lithographic printing plate, a printing ink, a toner, and a heat ray-shielding material can be provided. Further, a production method capable of synthesizing a naphthalocyanine compound at a high yield which has a specific structure and which is employed for the use described above is provided.

The invention is suitable for various uses such as identification of originality (for example, types or authenticity of goods), signal detection, prevention of counterfeiting, welding of a polymer, and shielding of infrared rays, by using absorption in the infrared region, especially in the electromagnetic spectrum of a near infrared region of from 750 nm to 950 nm, and particularly from 800 nm to 900 nm, and for use in materials employed for such uses (for example, an ink, mineral oil, a heat ray-shielding material, and a plate for lithographic printing).

### DESCRIPTION OF EMBODIMENTS

Hereinafter, the image detection method, the signal conversion method, the ink, the toner, the method of proving authenticity of goods or a support, the mineral oil, the heat ray-shielding material, the polymer welding method, and the method of producing a lithographic printing plate, of the invention are explained in detail.

Further, in the present specification, the symbol "-" is also used as the meaning including the lower limit and the upper limit of the numerical value described before and after the symbol.

### <Group described in the invention>

Herein, groups described in the invention are explained in greater detail before compounds are explained.
In the present specification, an aliphatic group means an alkyl group, a substituted alkyl group, an alkenyl group, a substituted alkenyl group, an alkynyl group, a substituted alkynyl group, an aralkyl group or a substituted aralkyl group. The alkyl- group may be branched and may form a ring. The alkyl group has preferably from 1 to 20 carbon atoms, and more preferably from 1 to 18 carbon atoms. An alkyl moiety of the substituted alkyl group may be the same as the above-mentioned alkyl group. The alkenyl group may be branched and may form a ring. The alkenyl group has preferably from 2 to 20 carbon atoms and more preferably from 2 to 18 carbon atoms. An alkenyl moiety of the substituted alkenyl group may be the same as the above-mentioned alkenyl group. The alkynyl group may be branched and may form a ring. The alkynyl group has preferably from 2 to 20 carbon atoms and more preferably from 2 to 18 carbon atoms. An alkynyl moiety of the substituted alkynyl group may be the same as the above-mentioned alkynyl group. An alkyl moiety of the aralkyl group and the substituted aralkyl group may be the same as the above-mentioned alkyl group. An aryl moiety of the aralkyl group and the substituted aralkyl group may be the same as the aryl group mentioned bellow.

Examples of the substituent group of the substituted alkyl group, the substituent group of the substituted alkenyl group, the substituent group of the substituted alkynyl group, and the substituent group of the alkyl moiety of the substituted aralkyl group include a halogen atom (such as a chlorine atom, a bromine atom, an iodine atom), an alkyl group [the alkyl group represents a linear, branched or cyclic, substituted or unsubstituted alkyl group, including an alkyl group (preferably an alkyl group having from 1 to 30 carbon atoms such as methyl, ethyl, n-propyl, isopropyl, t-butyl, n-octyl, eicosyl, 2-chloroethyl, 2-cyanoethyl, and 2-ethylhexyl), a cycloalkyl group (preferably a substituted or unsubstituted cycloalkyl group having from 3 to 30 carbon atoms such as cyclohexyl, cyclopentyl, and 4-n-dodecylcyclohexyl), a bicycloalkyl group (preferably a substituted or unsubstituted bicycloalkyl group having from 5 to 30 carbon atoms, that is, a monovalent group in which one hydrogen atom is removed from bicycloalkane having from 5 to 30 carbon atoms, for example, bicyclo [1,2,2]heptan-2-yl, and bicyclo[2,2,2]octan-3-yl), and a tricyclo structure further contains more ring structures. An alkyl group in substituent groups described hereinafter (for example, an alkyl group of an alkylthio group) means such a concept of the alkyl group], an alkenyl group [the alkenyl group represents a linear, branched or cyclic, substituted or unsubstituted alkenyl group, including an alkenyl group (preferably a substituted or unsubstituted alkenyl group having from 2 to 30 carbon atoms such as vinyl, allyl, prenyl, geranyl, and oleyl), a cycloalkenyl group (preferably a substituted or unsubstituted cycloalkenyl group having from 3 to 30 carbon atoms, that is, a monovalent group in which one hydrogen atom is removed from cycloalkene having from 3 to 30 carbon atoms, for example, 2-cyclopentene-1 -yl, and 2-cyclohexene-1-yl), a bicycloalkenyl groups (a substituted or unsubstituted bicycloalkenyl group, preferably a substituted or unsubstituted bicycloalkenyl group having from 5 to 30 carbon atoms, that is, a monovalent group in which one hydrogen atom is removed from bicycloalkene having one double bond such as bicyclo[2,2,1]hept-2-en-1-yl, and bicyclo[2,2,2]oct-2-en-4-yl)], an alkynyl group (preferably a substituted or unsubstituted alkynyl group having from 2 to 30 carbon atoms such as ethynyl, propargyl, and a trimethylsilylethynyl group),

an aryl group (preferably a substituted or unsubstituted aryl group having from 6 to 30 carbon atoms such as phenyl, p-tolyl, naphthyl, m-chlorophenyl, and o-hexadecanoylaminophenyl), a heterocyclic group (preferably a monovalent group in which one hydrogen is removed from a 5- or 6-membered, substituted or unsubstituted, aromatic or non-aromatic heterocyclic compound, more preferably a 5- or 6-membered aromatic heterocyclic group having from 3 to 30 carbon atoms such as 2-furyl, 2-thienyl, 2-pyrimidinyl, and 2-benzothiazolyl), a cyano group, a hydroxyl group, a nitro group, a carboxyl group, an alkoxy group (preferably a substituted or unsubstituted alkoxy group having from 1 to 30 carbon atoms such as methoxy, ethoxy, isopropoxy, t-butoxy, n-octyloxy, and 2-methoxyethoxy), an aryloxy group (preferably a substituted or unsubstituted aryloxy group having from 6 to 30 carbon atoms such as phenoxy, 2-methylphenoxy, 4-t-butylphenoxy, 3-nitrophenoxy, and 2-tetradecanoylaminophenoxy), a silyloxy group (preferably a silyloxy group having from 3 to 20 carbon atoms such as trimethylsilyloxy and t-butyldimethylsilyloxy), a heterocyclic oxy group (preferably a substituted or unsubstituted heterocyclic oxy group having from 2 to 30 carbon atoms, 1-phenyltetrazol-5-oxy and 2-tetrahydropyranyloxy), an acyloxy group (preferably a formyloxy group, a substituted or unsubstituted alkylcarbonyloxy group having from 2 to 30 carbon atoms, and a substituted or unsubstituted arylcarbonyloxy group having from 6 to 30 carbon atoms such as formyloxy, acetyloxy, pivaloyloxy, stearoyloxy, benzoyloxy, and p-methoxyphenylcarbonyloxy), a carbamoyloxy group (preferably a substituted or unsubstituted carbamoyloxy group having from 1 to 30 carbon atoms such as N,N-dimethylcarbamoyloxy, N,N-diethylcarbamoyloxy, morpholinocarbonyloxy, N,N-di-n-octylaminocarbonyloxy, and N-n-octylcarbamoyloxy), an alkoxycarbonyloxy group (preferably a substituted or unsubstituted alkoxycarbonyloxy group having from 2 to 30 carbon atoms such as methoxycarbonyloxy, ethoxycarbonyloxy, t-butoxycarbonyloxy, and n-octylcarbonyloxy), an aryloxycarbonyloxy group (preferably a substituted or unsubstituted aryloxycarbonyloxy group having from 7 to 30 carbon atoms such as phenoxycarbonyloxy, p-methoxyphenoxycarbonyloxy, and p-n-hexadecyloxyphenoxycarbonyloxy),

an amino group (preferably an amino group, a substituted or unsubstituted alkylamino group having from 1 to 30 carbon atoms and a substituted or unsubstituted anilino group having from 6 to 30 carbon atoms such as amino, methylamino, dimethylamino, anilino, N-methyl-anilino, and diphenylamino), an acylamino group (preferably, a formylamino group, a substituted or unsubstituted alkylcarbonylamino group having from 1 to 30 carbon atoms, and a substituted or unsubstituted arylcarbonylamino group having from 6 to 30 carbon atoms such as formylamino, acetylamino, pivaloylamino, lauroylamino, benzoylamino, and 3,4,5-tri-n-octyloxyphenylcarbonylamino), an aminocarbonylamino group (preferably a substituted or unsubstituted aminocarbonylamino group having from 1 to 30 carbon atoms such as carbamoylamino, N,N-dimethylaminocarbonylamino, N,N-diethylaminocarbonylamino, and morpholinocarbonylamino), an alkoxycarbonylamino group (preferably a substituted or unsubstituted alkoxycarbonylamino group having from 2 to 30 carbon atoms such as methoxycarbonylamino, ethoxycarbonylamino, t-butoxycarbonylamino, n-octadecytoxycarbonylamino, and N-methyl-methoxycarbonylamino), an aryloxycarbonylamino group (preferably a substituted or unsubstituted aryloxycarbonylamino group having from 7 to 30 carbon atoms such as phenoxycarbonylamino, p-chlorophenoxycarbonylamino, and m-n-octyloxyphenoxycarbonylamino), a sulfamoylamino group (preferably a substituted or unsubstituted sulfamoylamino group having from 0 to 30 carbon atoms such as sulfamoylamino, N,N-dimethylaminosulfonylamino, and N-n-octylaminosulfonylamino), an alkyl- and aryl-sulfonylamino group (preferably substituted or unsubstituted alkylsulfonylamino having from 1 to 30 carbon atoms and substituted or unsubstituted arylsulfonylamino group having from 6 to 30 carbon atoms such as methylsulfonylamino, butylsulfonylamino, phenylsulfonylamino, 2,3,5-trichlorophenylsulfonylamino, and p-methylsulfonylamino), a mercapto group,

an alkylthio group (preferably a substituted or unsubstituted alkylthio group having from 1 to 30 carbon atoms such as methylthio, ethylthio, and n-hexadecylthio), an arylthio group (preferably substituted or unsubstituted arylthio having from 6 to 30 carbon atoms such as phenylthio, p-chlorophenylthio, and m-methoxyphenylthio), a heterocyclic thio group (preferably a substituted or unsubstituted heterocyclic thio group having from 2 to 30 carbon atoms such as 2-benzothiazolylthio, and 1-phenyltetrazol-5-ylthio), a sulfamoyl group (preferably a substituted or unsubstituted sulfamoyl group having from 0 to 30 carbon atoms such as N-ethylsulfamoyl, N-(3-dodecyloxypropyl)sulfamoyl, N,N-dimethylsulfamoyl, N-acetylsulfamnoyl, N-benzoylsulfamoyl, and N-(N'-phenylcarbamoyl)sulfamoyl), a sulfo group, an alkyl- and aiyl-sulfinyl group (preferably a substituted or unsubstituted alkylsulfinyl group having from 1 to 30 carbon atoms and a substituted or unsubstituted arylsulfinyl group having from 6 to 30 carbon atoms such as methylsulfinyl, ethylsulfinyl, phenylsulfinyl, and p-methylphenylsulfinyl),

an alkyl- and aryl-sulfonyl group (preferably a substituted or unsubstituted alkylsulfonyl group having from 1 to 30 carbon atoms and a substituted or unsubstituted arylsulfonyl group having from 6 to 30 such as methylsulfonyl, ethylsulfonyl, phenylsulfonyl, and p-methytphenylsutfonyl), an acyl group (preferably a formyl group, a substituted or unsubstituted alkylcarbonyl group having from 2 to 30 carbon atoms, a substituted or unsubstituted arylcarbonyl group having from 7 to 30 carbon atoms, and a substituted or unsubstituted heterocyclic carbonyl group which has from 4 to 30 carbon atoms and is bonded to the carbonyl group by a carbon atom, such as acetyl, pivaloyl, 2-chloroacetyl, stearoyl, benzoyl, p-n-octyloxyphenylcarbonyl, 2-pyridylcarbonyl, and 2-furylcarbonyl), an aryloxycarbonyl group (preferably a substituted or unsubstituted aryloxycarbonyl group having from 7 to 30 carbon atoms such as phenoxycarbonyl, o-chlorophenoxycarbonyl, m-nitrophenoxycarbonyl, and p-t-butylphenoxycarbonyl), an alkoxycarbonyl group (preferably a substituted or unsubstituted alkoxycarbonyl group having from 2 to 30 carbon atoms such as methoxycarbonyl, ethoxycarbonyl, t-butoxycarbonyl, and n-octadecyloxycarhonyl), a carbamoyl group (preferably substituted or unsubstituted carbamoyl having from 1 to 30 carbon atoms such as carbamoyl, N-methylcarbamoyl, N,N-dimethylcarbamoyl, N,N-di-n-octylcarbamoyl, and N-(methylsulfonyl)carbamoyl),

an aryl- or heterocyclic- azo group (preferably a substituted or unsubstituted arylazo group having from 6 to 30 carbon atoms and a substituted or unsubstituted heterocyclic azo group having from 3 to 30 carbon atoms such as phenylazo, p-chlorophenylazo, and 5-ethylthio-1,3,4-thiadiazol-2-ylazo), an imido group (preferably N-succinimido and N-phthalimido), a phosphino group (preferably a substituted or unsubstituted phosphino group having from 2 to 30 carbon atoms such as dimethylphosphino, diphenylphosphino, and methylphenoxyphosphino), a phosphinyl group (preferably a substituted or unsubstituted phosphinyl group having from 2 to 30 carbon atoms such as phosphinyl, dioctyloxyphosphinyl, and diethoxyphosphinyl), a phosphinyloxy group (preferably a substituted or unsubstituted phosphinyloxy group having from 2 to 30 carbon atoms such as diphenoxyphosphinyloxy, and dioctyloxyphosphinyloxy), a phosphinylamino group (preferably a substituted or unsubstituted phosphinylamino group having from 2 to 30 carbon atoms such as dimethoxyphosphinylamino, and dimethylaminophosphinylamino), a silyl group (preferably a substituted or unsubstituted silyl group having from 3 to 30 carbon atoms such as trimethylsilyl, t-butyldimethylsilyl, and phenyldimethylsilyl).

Of those having a hydrogen atom in the above-mentioned functional groups, the hydrogen atom may be removed and may be substituted with any of the above-mentioned groups. Examples of such a functional group include an alkylcarbonylaminosulfonyl group, an arylcarbonylaminosulfonyl group, an alkylsulfonylaminocarbonyl group, and an arylsulfonylaminocarbonyl group. Specific examples thereof include a methylsulfonylaminocarbonyl group, a p-methylphenylsulfonylaminocarbonyl group, an acetylaminosulfonyl group, and a benzoylaminosulfonyl group.

Examples of a substituent group of the aryl moiety in the substituted aralkyl group include a substituent group of the substituted aryl group mentioned below.

In the present specification, the aromatic group means an aryl group or a substituted aryl group. The aromatic group may be condensed with an aliphatic ring, any other aromatic ring, or a heterocycle. The aromatic group has preferably from 6 to 40 carbon atoms, more preferably from 6 to 30 carbon atoms, and even more preferably from 6 to 20 carbon atoms. Of those, the aryl group is preferably a phenyl group or naphthyl group which may have a substituent group, and particularly preferably a phenyl group which may have a substituent group.

Examples of the substituent group of the substituted aryl group include such as those described hereinbefore for "the substituent group of the substituted alkyl group, the substituent group of the substituted alkenyl group, the substituent group of the substituted alkenyl group, and the substituent group of the alkyl moiety of the substituted aralkyl group."

In the present specification, the heterocyclic group contains at least one hetero atom as a ring-forming atom, and may be any one of saturated or unsaturated. The heterocyclic group may be an aromatic ring, may form a condensed ring with other rings, and may have a substituent group. Further, as the number of ring members, a 4- to 8-membered ring is preferable.

According to the invention, it is preferable that an aromatic 5-membered or 6-membered, saturated or unsaturated heterocycle is included. The heterocycle may be condensed with an aliphatic ring, an aromatic ring, or any other hetercycle. Preferred examples of the hetero atom of the heterocycle include B, N, O, S, Se and Te, and N,O and S are preferable as the hetero atom. It is preferable that the carbon atom of the heterocycle preferably has a free atomic valence (monovalent) (the heterocyclic group is bonded through the carbon atom). The heterocyclic group has preferably from I to 40 carbon atoms, more preferably from 1 to 30 carbon atoms, and even more preferably from 1 to 20 carbon atoms. Examples of the saturated heterocycle include a pyrrolidine ring, a morpholine ring, a 2-bora-1,3-dioxolane ring, and a 1,3-thiazolidine ring. Examples of the unsaturated heterocycle include an imidazole ring, a thiazole ring, a benzothiazole ring, a benzoxazole ring, a benzotriazole ring, a benzoselenazole ring, a pyridine ring, a pyrimidine ring, and a quinoline ring. The heterocyclic group may have a substituent group. Examples of the substituent group of the heterocyclic group include such as those mentioned hereinbefore for the substituent group of the substituted alkyl group, of the substituted alkenyl group, of the substituted alkynyl group and of the alkyl moiety of the substituted aralkyl group.

A infrared-absorbing agent used in the invention preferably has a spectroscopic absorption maximum wavelength of 730 nm, or more, more preferably 760 nm or more, and still more preferably 780 nm or more, from the viewpoint of spectroscopic absorption characteristics.

Examples of the compound which satisfies the spectroscopic absorption characteristics described above include a phthalocyanine compound, a cyanine compound, a squarylium compound, a diimmonium compound, a polymethine compound, an azomethine compound, an oxonol compound, a croconium compound and a dithiol metal complex compound. Examples thereof include such as those disclosed in JP-A Nos. 2000-281919, 10-180947, and 2003-139946. Among these, a phthalocyanine compound, a cyanine compound, a squarylium compound, a diimmonium compound, a polymethine compound, an oxonol compound, and a croconium compound are preferable, a phthalocyanine compound, a cyanine compound, a diimmonium compound, an oxonol compound, and a croconium compound are more preferable, a phthalocyanine compound, a diimmonium compound, an oxonol compound, and a croconium compound are still more preferable, a phthalocyanine compound, a diimmonium compound, and an oxonol compound are even still more preferable, and a phthalocyanine compound is the most preferable.

### (Compound represented by Formula (I))

A compound represented by Formula (I) used in the invention is explained in detail hereinbelow.

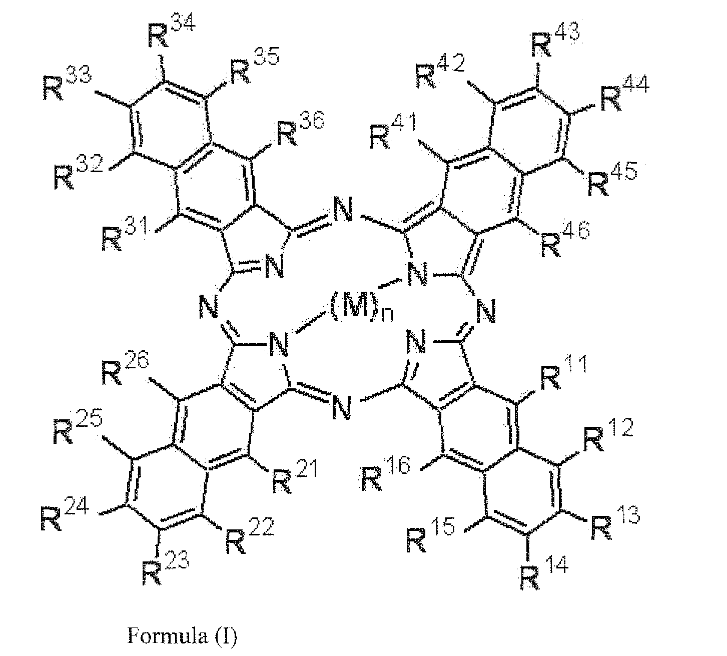

wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring. M represents a hydrogen atom, a metal ion, or a group containing a metal ion. n represents 1 or 2.

Examples of the substituent group for R¹¹ to R⁴⁶ include the above described substituent group of the substituted alkyl group, of the substituted alkenyl group, of the substituted alkynyl group, and of the alkyl moiety of the substituted aralkyl group.
A halogen atom, an alkyl- group, an alkenyl group, an alkynyl group, an aryl group, a cyano group, a hydroxyl group, a nitro group, a carboxyl group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group, an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, a sulfo group, an alkyl- or aryl-sulfinyl group, an alkyl- or aryl-sulfonyl group, an acyl group, an aryloxycarbonyl group, an alkoxycarbonyl group, a carbamoyl group, an imido group, a phosphino group, a phosphinyl group, a phosphinyloxy group, a phosphinylamino group, and a silyl group are preferable; a halogen atom, an alkyl group, an aryl group, a cyano group, a hydroxyl group, a nitro group, a carboxyl group, an alkoxy group, an aryloxy group, a silyloxy group, a heterocyclic oxy group, an acyloxy group, a carbamoyloxy group, an amino group, an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, a sulfo group, an alkyl- or aryl-sulfinyl group, an alkyl- or aryl-sulfonyl group, a carbamoyl group, an imido group, a phosphino group, a phosphinyl group, a phosphinyloxy group, a phosphinylamino group, and a silyl group are more preferable; a halogen atom, an alkyl group, an aryl group, a hydroxyl group, an alkoxy group, an aryloxy group, an amino group, a mercapto group, an alkylthio group, an arylthio group, a sulfamoyl group, a sulfo group, an alkyl- or aryl-sulfinyl group, and an alkyl- or aryl-sulfonyl group are even more preferable; a halogen atom, an alkyl group, an aryl group, an alkoxy group, an aryloxy group, an alkylthio group, and an arylthio group are still more preferable; a halogen atom, an alkyl group having from 1 to 20 carbon atoms, an aryl group having from 6 to 20 carbon atoms, an alkoxy group having from 1 to 20 carbon atoms, an aryloxy group having from 6 to 20 carbon atoms, an alkylthio group having from 1 to 20 carbon atoms, and an arylthio group having from 6 to 20 carbon atoms are further more preferable; an alkyl group having from 1 to 8 carbon atoms, an aryl group having from 6 to 10 carbon atoms, an alkoxy group having from 1 to 8 carbon atoms, an aryloxy group having from 6 to 10 carbon atoms, an alkylthio group having from 1 to 8 carbon atoms, and an arylthio group having from 6 to 10 carbon atoms are still further preferable; an alkoxy group having from 1 to 6 carbon atoms, an aryloxy group having from 6 to 8 carbon atoms, an alkylthio group having from I to 6 carbon atoms, and an arylthio group having from 6 to 8 carbon atoms are even further preferable; an alkoxy group having from I to 4 carbon atoms is even still preferable; and an n-butoxy group is most preferable.

Further, most preferably, each of R¹², R¹³, R¹⁴, R¹⁵, R²², R²³, R²⁴, R²⁵, R³², R³³, R³⁴, R³⁵, R⁴², R⁴³, R⁴⁴ and R⁴⁵ is a hydrogen atom.
Most preferably, each of R¹¹, R¹⁶, R²¹, R²⁶, R³¹, R³⁶, R⁴¹ and R⁴⁶ is an n-butoxy group.

As (M)ₙ, two hydrogen atoms, two Li⁺'s, two Na⁺'s, two K⁺'s, two Rb⁺'s, two Cs⁺'s, Be²⁺, Mg²⁺, Ca²⁺, Ti²⁺, Mn²⁺, Fe²⁺, Co²⁺, Ni²⁺, Cu²⁺, Zn²⁺, Ru²⁺, Rh²⁺, Pd²⁺, Pt²⁺, Ba²⁺, Cd²⁺, Hg²⁺, Pb²⁺, Sn²⁺, Al-Cl, Al-Br, Al-F, Al-I, Ga-Cl, Ga-F, Ga-I, Ga-Br, In-Cl, In-Br, In-I, In-F, Tl-Cl, Tl-Br, Tl-I, Tl-F, Mn-OH, Fe-Cl, Ru-Cl, CrCl₂, SiCl₂, SiBr₂, SiF₂, SiI₂, ZrCl₂, GeCl₂, GeBr₂, GeI₂, GeF₂, SnCl₂, SnBr₂, SnI₂, SnF₂, TiC1₂, TiBr₂, TiF₂, Si(OH)₂, Ge(OH)₂, Zr(OH)₂, Mn(OH)₂, Sn(OH)₂, TiR₂, CrR₂, SiR₂, SnR₂, GeR₂, Si(OR)₂, Sn(OR)₂, Ge(OR)₂, Ti(OR)₂, Cr(OR)₂, Sn(SR)₂, Ge(SR)₂ [where R represents an aliphatic group or an aromatic group]. VO, MnO, and TiO are preferable, two hydrogen atoms, two Li⁺'s, two Na⁺'s, two K⁺'s, two Rb⁺'s, Be²⁺, Mg²⁺, Ca²⁺, Ti²⁺, Mn²⁺, Fe²⁺, Co²⁺, Ni²⁺, Cu²⁺, Zn²⁺, Ru²⁺, Rh²⁺, Pd²⁺, Pt²⁺, Ba²⁺, Sn²⁺, Al-C, Al-Br, Ga-Cl, Ga-F, Ga-I, Ga-Br, In-Cl, In-Br, Tl-Cl, Tl-Br, Mn-OH, Fe-Cl, Ru-Cl, CrCl₂, SiCl₂, SiBr₂, ZrCl₂, GeC1₂, GeBr₂, SnCl₂. SnBr₂, TiCl₂, TiBr₂, Si(OH)₂, Ge(OH)₂, Zr(OH)₂, Mn(OH)₂, Sn(OH)₂, TiR₂, CrR₂, SiR₂, SnR₂, GeR₂, Si(OR)₂, Sn(OR)₂, Ge(OR)₂, Ti(OR)₂, Cr(OR)₂, Sn(SR)₂, Ge(SR)₂ [where R represents an aliphatic group or an aromatic group], VO, MnO, and TiO are more preferable, and two hydrogen atoms, two Li⁺'s, two Na⁺'s, two K⁺'s, Be²⁺, Mg²⁺, Ca²⁺, Ti²⁺, Mn²⁺, Fe²⁺, Co²⁺, Ni²⁺, Cu²⁺, Zn²⁺, Ru²⁺, Rh²⁺, Pd²⁺, Pt²⁺, Ba²⁺, Sn²⁺, Al-Cl, Ga-Cl, In-Cl, Tl-Cl, Mn-OH, Fe-Cl, Ru-Cl, CrC1₂, SiC1₂, ZrCl₂, GeCl₂. TiCl₂, Si(OH)₂, Ge(OH)₂, Zr(OH)₂, Mn(OH)₂, TiR₂, CrR₂, SiR₂, GeR₂, Si(OR)₂, Ge(OR)₂, Ti(OR)₂, Cr(OR)₂ [where R represents an aliphatic group or an aromatic group], VO, MnO, and TiO are even more preferable. Even still more preferably, M¹ is two hydrogen atoms, Mn²⁺, VO, Zn²⁺ or Cu²⁺, even further still more preferably VO, Zn²⁺ or Cu²⁺, and most preferably Cu²⁺.

The bond between M and each of the two secondary nitrogen atoms of the naphthalocyanine ring may be any of a covalent bond, an ionic bond, and a coordination bond. When M is a transition metal, M may further form a coordination bond with two tertiary nitrogen atoms of the naphthalocyanine ring.

Hereinafter, specific examples of the compound represented by Formula (I) of the invention are shown, but the invention is not limited thereto.

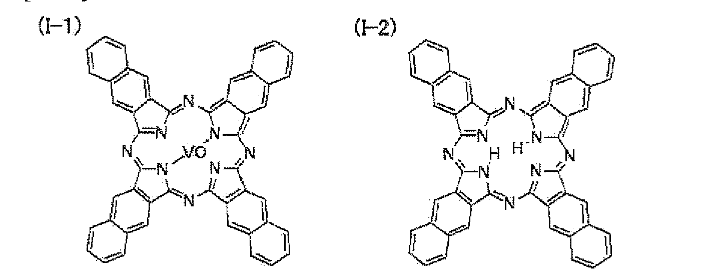

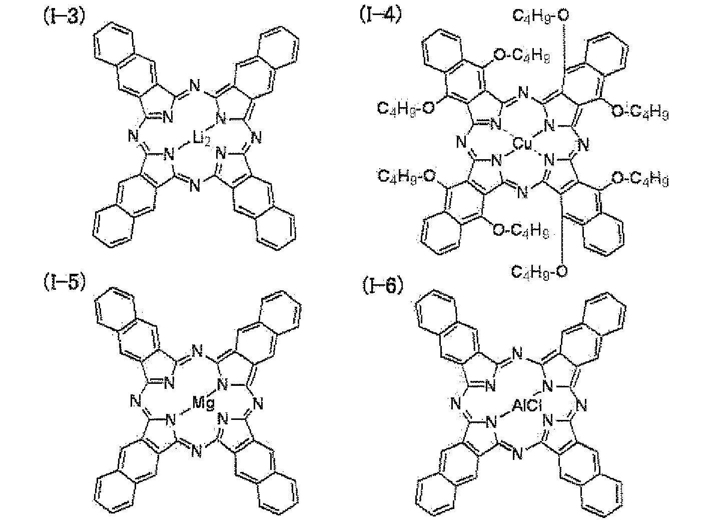

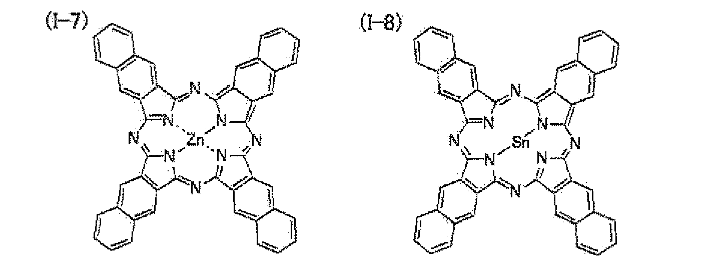

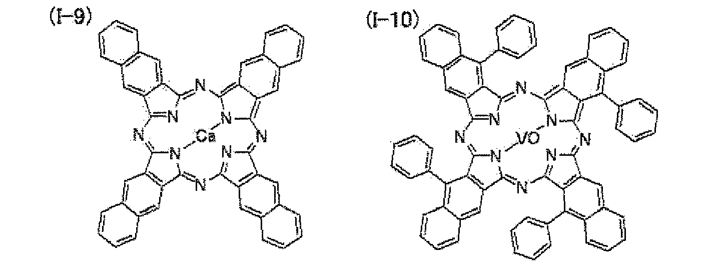

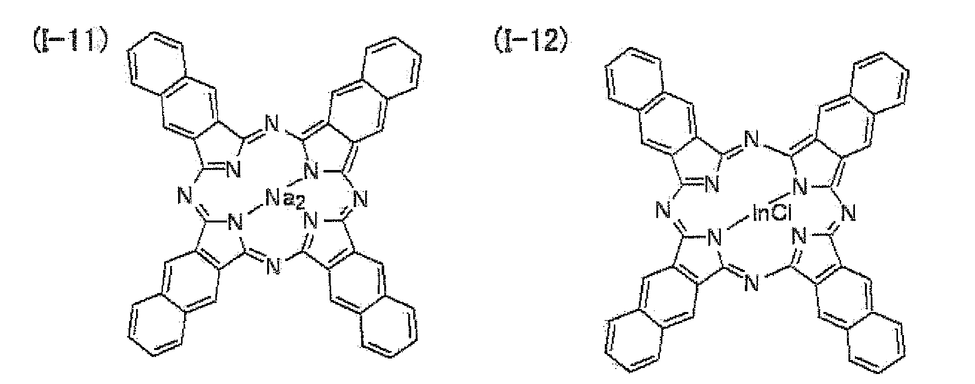

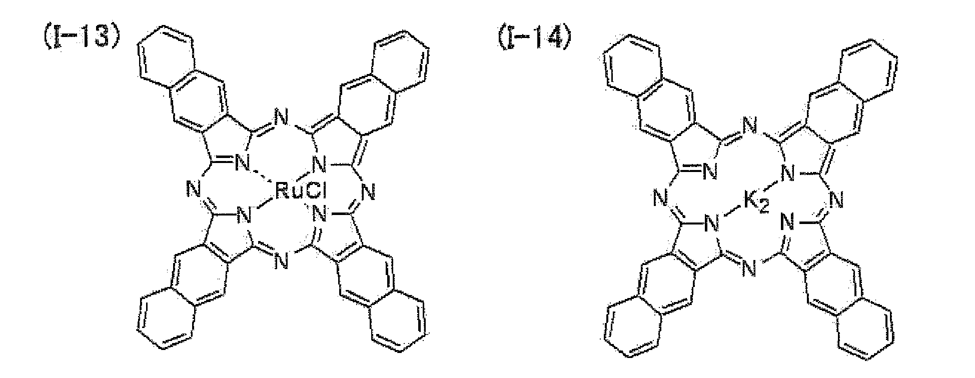

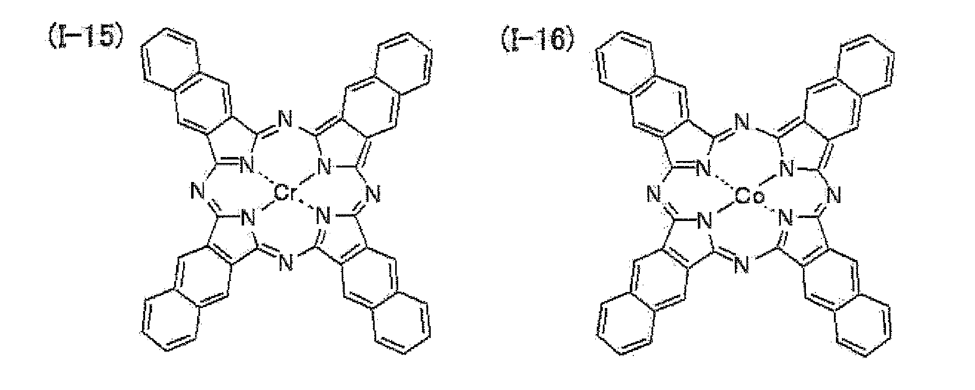

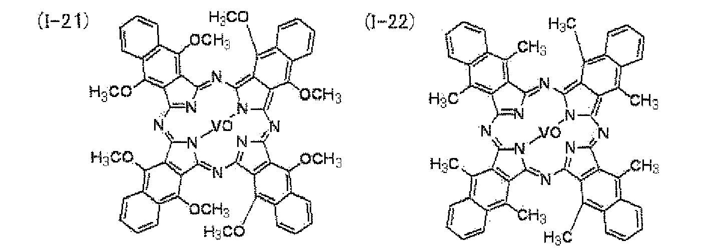

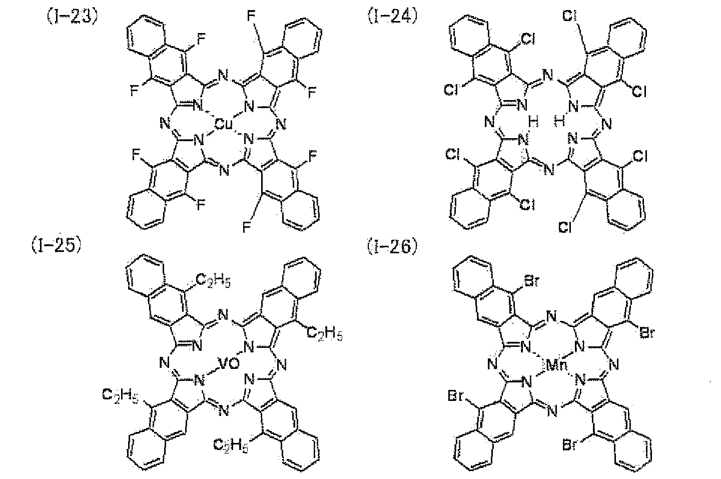

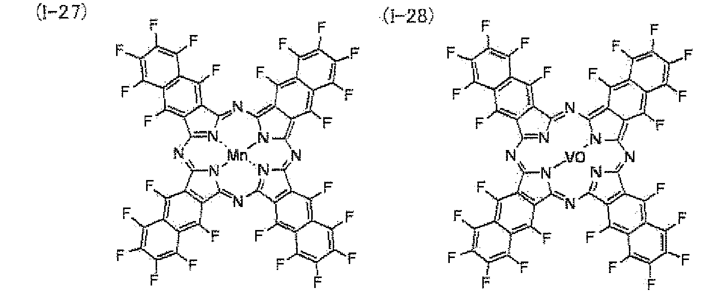

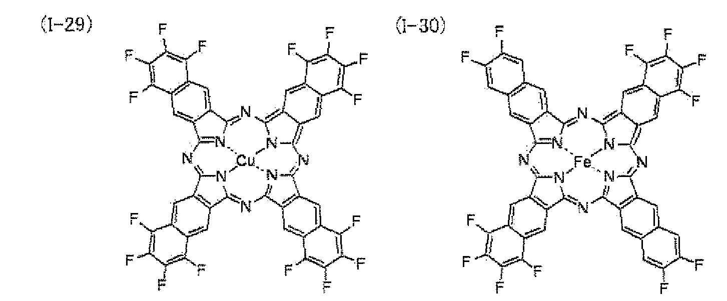

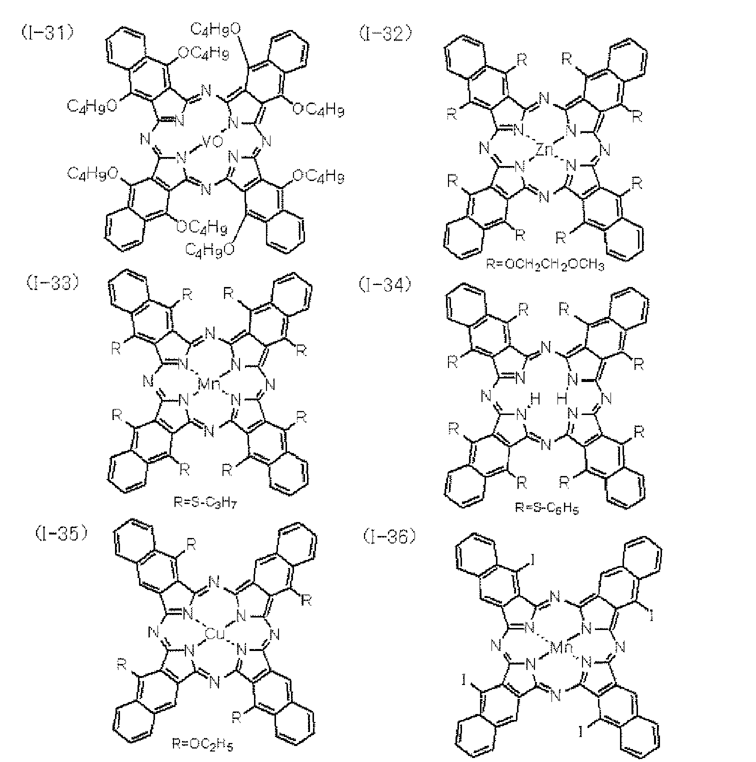

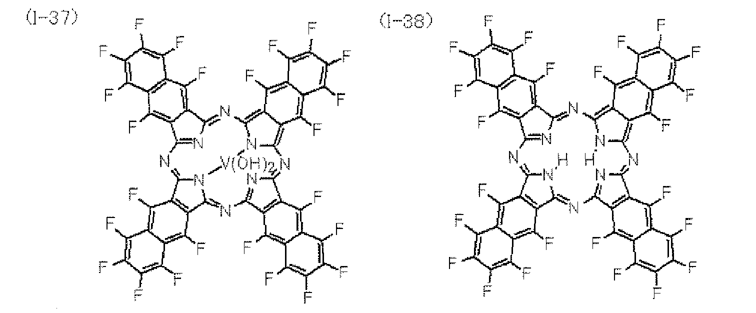

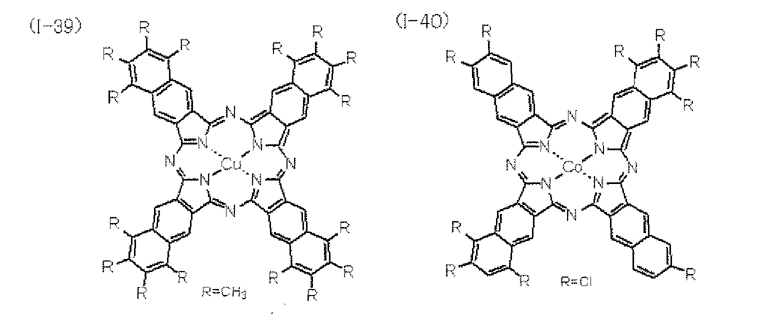

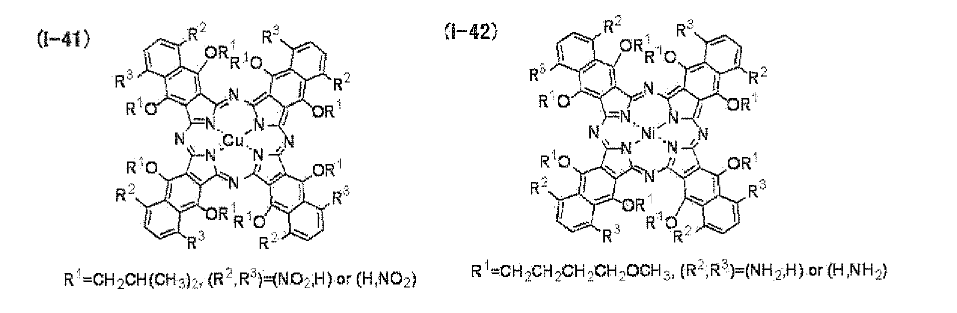

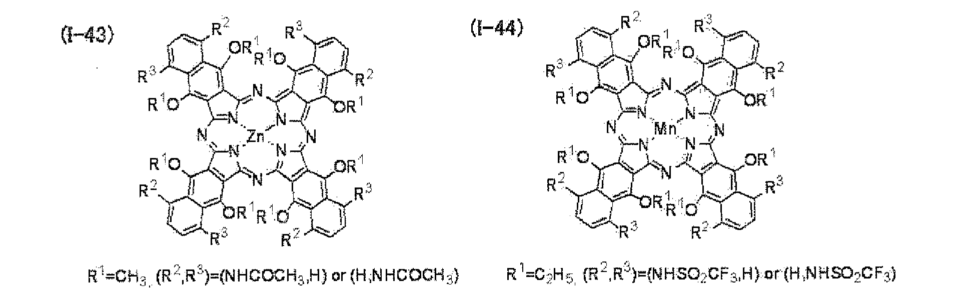

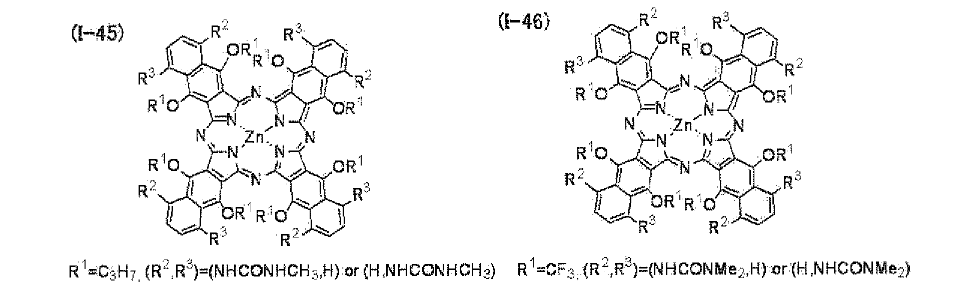

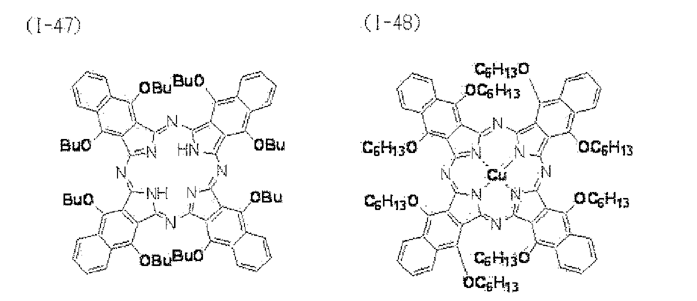

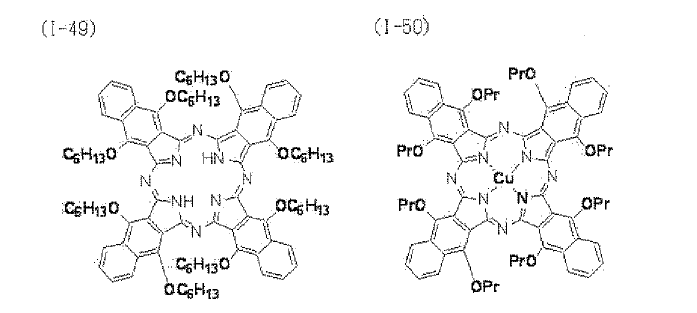

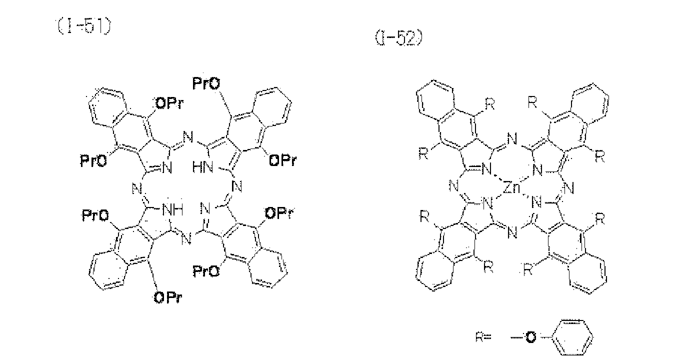

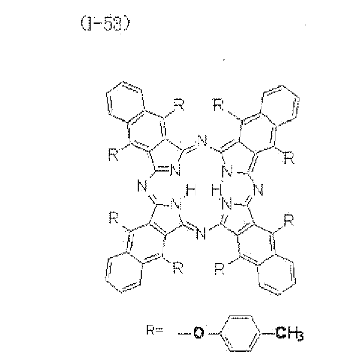

The method of producing the compound of Formula (I) of the present invention includes, for example, a process of using t-BuOM³ and lithium halide in a single solvent or a mixture of plural solvents.
In the method of producing the compound of Formula (II) of the present invention, a solvent represented by R¹OH- R⁸OH may be used.

A preferred amount of t-BuOM³ is, with respect to the naphtalene compound as a raw material, 0.1 mole ratio to 20 mole ratio, preferably 1 mole ratio to 8 mole ratio, and more preferably 2 mole ratio to 4 mole ratio. A preferred amount of the lithium halide is, with respect to the naphthalene compound as a raw material, 0.1 mole ratio to 50 mole ratio, preferably 1. mole ratio to 15 mole ratio, and more preferably 3 mole ratio to 10 mole ratio.

An amount of the solvent represented by R¹OH-R⁸OH is, with respect to the naphthalene compound as a raw material, 0.1 mole ratio to 50 mole ratio, preferably 1 mole ratio to 15 mole ratio, and more preferably 2 mole ratio to 6 mole ratio. It is also preferable that an amide solvent (for example, N,N-dimethylformamide, N,N-dimethylacetamide, and I -methyl-2-pyrrolidone), a sulfone solvent (for example, sulfolane), a sulfoxide solvent (for example, dimethyl sulfoxide), an ether solvent (for example, dioxane and cyclopentyl methyl ether), a ketone solvent (for example, acetone and cyclohexanone), a hydrocarbon solvent (for example, toluene and xylene), a halogen-containing solvent (for example, tetrachloroethane and chlorobenzene), or a pyridine solvent (for example, pyridine, γ-picoline, and 2,6-lutidine) is further mixed with these solvents described above.

Preferably, the method of producing the compound of Formula (I) of the present invention further includes a process of using hydroquinone.
A preferred amount of hydroquinone is, with respect to the naphthalene compound as a raw material, 0.05 mole ratio to 5 mole ratio, preferably 0.1 mole ratio to 3 mole ratio, and more preferably 0.2 mole ratio to 2 mole ratio. It is preferable that addition of hydroquinone may be carried out at the time of adding the naphthalene compound as a raw material, lithium halide and t-BuOM³, at the time after the completion of temperature increase, or any time between the initiation of the reaction and right before the termination of the reaction.

The reaction temperature is from -30°C to 250°C, preferably from 0°C to 200°C, more preferably from 20°C to 150°C, and still more preferably from 50°C to 120°C. The reaction time is preferably within the range of from 5 min to 30 hours.

The compound represented by Formula (I) of the invention is particularly useful as Infra-red Absorbers (IRA) in use described below [(a) to (c) described below].

### (a) Heat Input System

It is useful for a system in which heat input is preferably performed at a specific position by exposure to infrared rays, for example, an electrophotography system using a toner containing the IRA in which laser-induced fixation of a latent toner image (latent image) is promoted by the presence of IRA; a printing plate manufacture system using a lithographic printing plate having a photosensitive layer which contains IRA in which the formation of a laser-induced image on the printing plate is promoted by the presence of the IRA in the photosensitive layer; a polymer welding system in which generation of laser-induced heat in the vicinity of the welding is promoted by the presence of the IRA, and the like.

According to the invention, when a lithographic printing plate is produced, a photosensitive layer containing the compound represented by Formula (I) is irradiated with an infrared laser in accordance with pattern information. By containing the compound represented by Formula (I), infrared rays (preferably the electromagnetic spectrum of near infrared rays of from 750 nm to 1500 nm, and in particular from 800 nm to 1000 nm) absorption efficiency is high and reduction in sensitivity due to deterioration of infrared absorption efficiency over time is suppressed.

Further, in welding polymer according to the invention, the compound represented by Formula (I) is used, and a polymer material is irradiated with an infrared laser at a region at which a welding is preferably formed. Here, the polymer material contains the compound represented by Formula (I). The polymer material is coated or printed with the compound that is preferable to form a welding (the compound represented by Formula (I))or there is provided a layer or film which contains the compound represented by Formula (I) and which is disposed adjacent to the polymer material that is preferable to form a welding. The polymer material may contain the compound represented by Formula (I) by extruding the compound into the polymer material. The compound represented by Formula (I) may be incorporated into the film by extrusion or solution deposition. By utilizing the compound represented by Formula (I), in the case of irradiation of infrared rays, the compound absorbs the irradiated infrared rays and then heat is well-generated, whereby favorable welding between the polymers can be performed while the welding can be performed easily and stably over a long period of time.

### (b) IR Radiation Protection System

It is useful for a system that is suitably used to absorb infrared rays from incident light for the protection against the effects of the IR irradiation, for example, heat resistant solar glazing for buildings or cars, sun visors, goggles for welding, and the like.

The heat ray-shielding material of the invention contains the compound represented by Formula (1). Examples of the shielding material include heat resistant solar glazing, sun visors, and goggles for welding, as described above. For example, by incorporating the IRA represented by Formula (I) into the glazing or a layer forming a part of the glazing, the interior of a glazed structure can be protected against the heating effect when an incident IR is irradiated since the IRA has excellent heat resistance and wet heat resistance.

In a sun visor, goggles for welding or the like, by incorporating the IRA represented by Formula (I) into a protective film or a layer forming a part of the protective film, attenuation of the IR irradiation passing through the light-permeable protective film (such as a visor, an eyepiece of goggles, or the like) can be achieved. In such a case, since the IRA represented by Formula (I) has excellent light resistance, heat resistance and wet heat resistance, an attenuation effect of the IR can be maintained for a long period of time.

### (c) Detection/Handling System

It is useful for a system used for, for example, tracing the movement of a drug doped with the IRA, enhancing a thermal signal in infrared ray imaging, fault detection, for example, detecting cracks by impregnation in a solution of the IRA, and automated detection and/or handling of products marked with the IRA, for example, mail sorting or in machine reading of prices and related information in supermarket operations and the like, and providing or enhancing a machine-detectable IR or heat energy signal for use in a medical treatment.

The image detection method of the invention includes detecting an image attached to a surface of goods or a support by irradiating with infrared rays and scanning, wherein the image contains the compound represented by Formula (I). In the invention, "goods" means a commercial product by itself, and "support" means a part or accessory of goods which is not a commercial product by itself (for example, price tag, a label, and the like).
According to the invention, by infrared-scanning, with an infrared detector, an image containing the compound represented by Formula (I) as an IRA, an image attached to the surface of goods or a support (superficial image) as a sample to be tested is detected. By containing the compound represented by Formula (I), absorption efficiency of infrared rays, preferably the electromagnetic spectrum of near infrared rays of from 750 nm to 1500 nm, and in particular from 800 nm to 1000 nm, is high while decrease in detection accuracy due to deterioration of infrared absorption efficiency over time is suppressed.

In an example of image formation, a temporary toner image is fixed on goods or a support by using a toner containing an IRA represented by Formula (I) and/or an IR-readable permanent toner image can be provided. Further, in order to provide the toner image, the permanent toner image can be formed on goods or a support by using an electrophotographic device including an IR supply source.

According to the signal conversion method of the invention, a thermal signal is converted into an enhanced thermal signal or a light signal is converted into a thermal signal by using the compound represented by Formula (I). According to the invention, when a thermal signal is obtained by irradiation with infrared rays, the thermal signal from a process, a product, or the like can be enhanced by incorporating the compound represented Formula (I) as an IRA into or onto the process medium or product from which the thermal signal is derived (image enhancing system).

By applying, to a solid, a liquid containing the compound represented by Formula (I) as the IRA and examining the solid about the presence of IRA therein, the solid can be detected by allowing IR to pass though an IR permeable solid.

It is particularly preferable for use in the automated recognition and handling and/or sorting of goods, such as mails and supermarket goods. By attaching, on goods or a support, an image which contains the IRA represented by Formula (I), it is possible to recognize, handle and/or sort target goods by machine reading a reflective signal generated when the image is irradiated with infrared rays and scanned. By containing the compound represented by Formula (I) as an IRA, absorption efficiency of infrared rays, preferably the electromagnetic spectrum of near infrared rays in from 750 nm to 1500 nm, and in particular frog 800 nm to 1000 nm is high and decrease in detection accuracy according due to deterioration of infrared absorption efficiency over time is suppressed.

When an IRA is applied to a surface of the goods or support in the above applications, application can be performed by dissolving or dispersing IRA into a liquid medium so as to form an IRA-containing liquid and applying the IRA-containing liquid to the surface by coating, or a discharging and printing method using an inkjet method.

### <Ink and Toner>

As a liquid containing IRA, ink containing the compound represented by Formula (I) may be used, for example.

The ink may have a configuration in which a colorant is contained. An ink in which both a colorant and IRA are dissolved in an ink medium is generally suitable for ink jet recording using an absorbent material such as paper, a card, or the like.

The ink may be configured to be photocurable by further using an alkoxylated or polyalkoxylated acrylate monomer and a photoinitiator, in addition to the above.

The compound represented by Formula (1) may be used preferably for a printing process or electrophotographic process using an ink or toner on a part or whole surface of goods or support, particularly to form a security marking or a label. In such a case, the compound represented by Formula (I) can be included in an ink, toner, or the like used for printing or electrophotography. The ink, toner, or the like may be configured so as to contain, in addition to the compound represented by Formula (I), a component generally used for an ink, toner, or the like.

Such a ink or toner containing the compound represented by Formula (I) can be used in security printing applications for detecting a counterfeit or fraud as described below. The method of printing is preferably selected from the following: offset printing, gravure printing, ink jet recording, intaglio printing, and anastatic printing. The compound represented by Formula (I) may also be used in an electrophotographic toner, a matrix or daisy-wheel printer ink and a non-impact printing method.

The compound represented by Formula (I) is preferably used for a method of proving authenticity of goods or a support. Specifically, the method of proving authenticity of goods or support of the invention includes a process of attaching, to goods or support, a marking (formed by an ink or the like) containing the compound represented by Formula (I) (security marker (a marker for security assurance)) by a variety of methods, for example, printing methods, irradiating the attached marking with infrared rays to detect and/or measure specific absorption. In this process, infrared absorption by the compound represented by Formula (I) is detected and measured, whereby identification of originality (for example, a type or authenticity of goods) and detection and prevention of counterfeiting can be performed. By using the compound represented by Formula (I), detection and measurement accuracy is excellent, and detection or the like can be performed stably for a long period of time.

Examples of the support used in the invention include, generally paper and rag paper; and preferably currency grade paper, plastics-coated paper, or laminated paper, and plastics (for example, bankcard-grade PVC), or plastic paper (for example, non-woven plastic paper).

Examples of goods of the invention include documents, packaging or goods having a printed marking such as banknotes, banknote thread, currency, travelers' checks, bonds, certificates, stamps (revenue stamps), lotteries, ownership documents, passports, identification cards, credit cards, charge cards, access cards, smart cards, brand authentication labels and tags, and tamperproof labels.

The authenticity of goods can be proven and established by attaching a marking to the goods or support with the compound represented by Formula (I) and detecting and/or measuring a specific absorption by the marking at the time of irradiation of infrared rays by, for example, a standard spectroscopic method.

The compound represented by Formula (I) can be used when it is preferable to provide a machine-detectable IR identification number or actuation signal, for example in valuable products such as documents, currency, jewelry, bonded materials and the like; in fuel and alcoholic drinks; and in a security marking such as computer controlled locks, alarms, and the like.

### Hereinafter, the applications (a) - (c) are explained in further detail.

### (a) Heat Input System

### i. Toner

Preferable examples of a toner include a flash fixing toner containing a binder resin, a colorant, and the IRA represented by Formula (I). Here, the content of IRA represented by Formula (I) is preferably about 0.01 to 5.0% by mass (more preferably 0.1 to 3.0% by mass), with respect to the total amount of the toner. The IRA is suitably dispersed (or dissolved) in the binder resin forming the matrix of toner particles.

The binder resin may be an appropriate resin suitably used in flash fixing toners, examples thereof include such as polystyrenes; copolymers of styrene with (meth)acrylic ester, acrylonitrile, or maleic ester; poly(meth)acrylic esters; polyesters; polyamides; epoxy resins; phenolic resins; hydrocarbon resins; and petroleum resins. Therese may be used alone or in combination with other resins or with an additive. Preferred examples of the binder resin include a polyester resin and epoxy resin of Bisphenol A and epichlorohydrin.

The colorant may be an appropriate colorant suitably used in the flash fixing toner, and examples thereof include various pigments or colorants such as chrome yellow, cadmium yellow, yellow iron oxide, titanium yellow, naphthol yellow, Hanza yellow, pigment yellow, benzidine yellow, permanent yellow, quinoline yellow, anthrapyrimidine yellow, permanent orange, molybdenum orange, vulcan fast orange, benzidine orange, indanthrene brilliant orange, iron oxide, amber, permanent brown, rose iron oxide red, antimony powder, permanent red, fire red, brilliant carmine, light fast red toner, permanent carmine, pyrazolone red, Bordeaux, helio-Bordeaux, rhodamine lake, DuPont oil red, thioindigo red, thioindigo maroon, watching red strontium, cobalt purple, fast violet, dioxane violet, methyl violet lake, methylene blue, aniline blue, cobalt blue, cerulean blue, chalco oil blue, nonmetal phthalocyanine blue, phthalocyanine blue, ultramarine blue, indanthrene blue, indigo, chrome green, cobalt green, pigment green B, green gold, phthalocyanine green, malachite green oxalate, and polychromo-bromo copper phthalocyanine.
The amount of colorant can be selected from a wide range but the amount is preferably in a range of from 3 parts by mass to 5 parts by mass, with respect to 100 parts by mass of the binder resin.

The flash fixing toner may contain other components such as a wax, a charge control agent, and/or a flow-enhancer.
The wax may be a polyolefin type or natural wax such as carnauba wax, montan wax, or a natural paraffin, polyethylene, polypropylene, polybutylene, an ethylene-propylene copolymer, an ethylene-butene copolymer, an ethylene-pentene copolymer, an ethylene-3-methyl-1-butene copolymer, and a copolymer of olefins and other monomers such as vinyl esters, halo-olefins, (meth)acrylic esters, or (meth) acrylic acid or derivatives thereof. The weight-average molecular weight of the wax is preferably from 1,000 to 45,000 daltons.

Examples of the charge control agent include nigrosine, a monoazo dye, zinc, hexadecyl succinate, alkyl ester or alkyl amide of naphthoic acid, nitrohumic acid, N,N-tetramethyl diamine benzophenone, N,N-tetramethyl benzidine, triazines and a metal complexe of salicylic acid. When the colorant is other than black, it is preferred that the charge control agent is substantially colorless.

Example of the flow-enhancer include fine particles of an inorganic substance such as colloidal silica, hydrophobic silica, hydrophobic titania, hydrophobic zirconia, and talc; and fine particles of an organic substance such as polystyrene beads and (meth)acrylic acid beads.

When the IRA is soluble or dispersible in the binder resin, a coupling agent and IRA (and other appropriate components mentioned above) are preferably compounded and kneaded together. After cooling and then pulverizing the resultant mixture, the particles are sorted.

### ii. Chemically Produced Toner

The IRA represented by Formula (I) may be mixed with a chemically produced toner and used. The toner may contain one or more preferable polymers, or mixtures of polymers when the molecular weight distribution and the melt rheology properties of the toner are to be controlled by using polymers with various molecular weights as the binder resin.

Examples of the suitable polymer include styrene-acrylate copolymers, styrene-butadiene copolymers, polyesters and hydrocarbon resins. The toner containing a colorant provides a colored image on the printed support or the charge control agent to promote incorporation of an electrical charge and a wax so as to have a tendency to be released from a fusion roller. Here, examples of the colorant include pigment (which includes a magnetic pigment and which may be a pigment that does not interfere with the absorption of infrared rays by the IRA) and a dye. Examples of the charge control agent include a metal complex such as complexes of Zn, Al, Fe or Cr, and polymeric materials such as phenolic polymers. Examples of the wax include hydrocarbon wax such as paraffin, polyethylene or polypropylene wax, wax derived from carbon monoxide and hydrogen such as Fischer-Tropsch wax, natural product wax such as carnauba wax, and synthetic wax such as ester or amide wax. The toner may also contain a surface additive such as silica, titania, alumina or polymeric particles, thereby adjusting fluidity, charging performance or transfer properties.

### iii. Offset Printing Plate

An offset printing plate is generally prepared from a recording material including a support and a photosensitive layer formed on a surface of the support, and a desired pattern can be formed on the photosensitive layer by irradiation of infrared rays. As an example of the recording material, there is a recording material which has a photosensitive layer containing an IRA and in which a desired pattern is formed on the photosensitive layer by irradiating with IR radiation, generally from a laser, in accordance with predetermined pattern information. In the method of producing the lithographic printing plate of the invention, the compound represented by Formula (I) is used as the IRA contained in the photosensitive layer. The compound represented by Formula (I) is suitable for a recording material in which a pattern formation is performed by using an IR laser having a main emission peak in a wavelength range of from 750 nm to 1000nm, such as a commercially available solid-state laser diode having a main radiation peak at about 830 nm. Examples of the recording material for an offset printing plate include such as those described in U.S. Patent No. 6,294,298 and the publications recited in the present specification; however, the invention is not limited thereto.

### iv. Laser Welding

The polymer welding method of the invention relates to laser welding of a polymer and the compound represented by Formula (I) is used for welding.

An example of laser welding is described in United Kingdom Patent (GB-A) No. 2,276,584, wherein there is provided a method of welding two bodies, in which in the two bodies of a thermoplastic material (one of which is substantially transparent to infrared ray (IR) irradiation and the other is substantially opaque to the infrared ray irradiation) that are held on each of two adjacent surfaces in contact (a contact region between the two bodies is clearly defined), the surface of the opaque body in the contact region is irradiated with infrared ray radiation through the transparent body to fuse the surfaces of the both bodies in the contact region together, whereby the surfaces of the both bodies are heated to a temperature at which they are bonded together by infrared absorption by the infrared-opaque body at the contact region and by transmission of generated heat.

The infra-red opacity of the opaque thermoplastic body can be enhanced by the incorporation of the compound represented by Formula (I) of the invention.

By using the method above, an infrared ray opaque body (thermoplastic pipe) can be radially reinforced by winding one or more layers of an infrared ray transparent body (fiber reinforced thermoplastic tape or film) around the pipe. In order to balance torsional force, it is convenient that a pair of two tapes which are the thermoplastic tape and a reverse tape thereof is wound on the pipe when the pipe is conveyed under pressure. Each tape is wound under such a tension that the tape and the pipe in close contact during the subsequent welding stage, in order to ensure that a welding is formed between them. The first tape in the pair (one pair including two) is applied directly to the pipe and the second tape is applied on top of the first tape under sufficient tension to hold both tapes firmly against the pipe such that the second tape is fused thereto in the welding stage. Further each of tapes or a pair of tapes can be wound on top of the first pair of tapes to provide additional reinforcement. Generally, each tape is formed from polyethylene and contains a number of reinforcing fibers that extend lengthwise of the tape and that is evenly distributed across the width the tape. Each fiber is conveniently formed from a bundle of fine filaments of a suitable reinforcing material, such as aramid. Alternatively, the pipe may be longitudinally reinforced by applying the reinforcing tape along the length of the pipe so that the strength of the tape is parallel to the longitudinal axis of the tape.

In any of these examples, the fiber reinforced thermoplastic tape forms substantially the IR-transparent body, the thermoplastic pipe forms the IR-opaque body and the fusion welding part is formed by irradiating the pipe with IR from the outside, through the tapes, in order to fuse the layers of thermoplastic material adjacent to the contact layer between the inner surface of the tape and the outer surface of the pipe.

The compound represented by Formula (I) of the invention may be used as a radiation absorbing additive in other known IR laser welding methods.

### (b) IR Radiation Protection System

### i. Heat Resistant Glazing

The compound represented by Formula (I) may be used as a wavelength gap-filling component (WGFC) of an optical body containing one of the following 1. and 2., for example, such as those described in U.S. Patent No. 6,049,419.

1.
   (a) a birefringent dielectric multilayer film, which may be a polarizer, mirror, or both, having a reflection band positioned such that an infrared ray of at least one polarization at a normal incident angle with respect to the film is reflected (herein, the reflection band has a short wavelength bandedge λₐ0 and a long wavelength bandedge λ_{b}0, at a normal incident angle, and a short wavelength bandedge λaθ and a long wavelength bandedge λ_{b}θ at a maximum usage angle θ, wherein λaθ is less than λₐ0, and λₐ0 is selectively positioned, at a wavelength greater than about 700 nm), and
   (b) a component which at least partially absorbs or reflects radiation in the wavelength region between λₐθ and λₐ0 at a normal angle of incidence (WGFC).

2.
   (a) an isotropic dielectric multilayer film having a reflection band positioned such that an infrared ray of at least one polarization at a normal incident angle with respect to the film is reflected (herein, the reflection band has a short wavelength bandedge λₐ0 and a long wavelengths bandedge λ_{b}0, at a normal incident angle, and a short wavelength bandedge λaθ and a long wavelength bandedge λ_{b}θ, at a maximum usage angle θ, wherein λaθ is less than λₐ0, and λₐ0 is selectively positioned at a wavelength greater than about 700 nm), and

(b) a component which at least partially absorbs or reflects radiation in the wavelength region between λₐθ and λₐ0 at a normal angle of incidence (WGFC).
   The optical body provides excellent reflectivity in the infrared region of the spectrum and excellent shading coefficient at a normal incident angle while still transmitting a visible light at all preferable incident angles.

The WGFC functions to absorb or reflect the infrared wavelengths that are not reflected at normal angles because of the need to shrift the reflection band of the film to higher wavelengths in order to minimize color changes detected at non-normal incidence. There is a possibility that the WGFC does not function at non-normal angles because the reflection band shifts to lower wavelengths depending on the position of the WGFC with respect to the film, preferably by being commensurate with the wavelength of the absorption or reflection of the WGFC. The WGFC may be incorporated into one or more of the film layers or incorporated into a separate or discrete part of the optical body, that is, a separate layer derived from the film (a) that can be attached thereto by lamination. In this type, the WGFC (that is, IRA) is incorporated into a separate layer attached to the film (a). The WGFC may be a part of the film or separate from the film, depending on the characteristics of the film to be combined with.

The film (a) and the WGFC (b) may be combined so that the film is placed on a surface nearest the sun as to be practical because it is more efficient for the film (a) and the WGFC (b) to reflect solar energy than to absorb the solar energy. If it is possible, it is preferable that the sunlight first encounters the film and then the WGFC. In a combined pane or two-ply perspex, the most preferable position of the film is the exterior nearest the sun, the next preferable position is between the panes or plies. The film may be placed on the interior surface; however that causes absorption of sunlight by the glass and then the light reaches the film, whereby a part of the light reflected from the film is absorbed. It may be preferable to position the film away from the sun; however, the part of the light can be absorbed by a component that is less sensitive to UV, whereby this arrangement is preferable when considered from a UV protection standpoint.

The WGFC (IRA) can be applied a surface of the film (a), to in a layer of glass or polymer such as polycarbonate, acrylic polymer or the like which is laminated to the film, or can be mixed in at least one of polymer layers of the film. From the viewpoint of a solar energy, the reflection band of the film shifts to lower wavelengths when the sun is at a high angle to be substantially commensurate with the λₘₐₓ region of the dye, whereby the WGFC is preferably on the innermost surface of the film (that is, a position away from the sun toward the interior). In this case, it is preferred reflecting the solar energy away from the film rather than absorbing the solar energy. The amount of the WGFC used in the optical body may be varied depending on the specific nature of IRA and the end use application. Generally, when applying to the surface of the film, the IRA is present on the surface at a proper concentration and coating thickness to accomplish the desired infrared absorption and an appearance. Generally, when the IRA is within an additional layer or within the multilayer optical body, the concentration is preferably in a range of from about 0.05% to about 0.5% with respect to the total mass of the optical body. It is highly desirable that the IRA is finely pulverized to such an extent that the particle size is less than the wavelength of the incident light. When the IRA is non-polar solvent-soluble and is heat stable, it can be coated or mixed with a solid plastic pellet and extruded.

However, the IRA represented by Formula (I) is not limited to the use only in an optical body of this type, but may be used in any other optical body having the same purpose, that is, attenuation of IR radiation.

### (c) Detection/Handling System

According to the image detection method of the invention, the compound represented by Formula (I) is contained in an image, and the image attached to the surface of the goods is detected by irradiating the image with an infrared ray and scanning. An infrared signal or a thermal signal is detected, or the strength of an infrared signal or thermal signal, from the image attached to the product to be detected (a thermal signal enhanced by the presence of an IRA in the image) can be measured through the compound represented by Formula (I). Hereby, a data handling for stock recording or accounting, an automated handling for manipulation of the products in accordance with signal information, or the like can be carried out.

### i. Image Enhancement System

The IRA is contained in order to enhance the strength of a thermal signal. One of the methods of producing and detecting a thermal energy difference between a material and its surroundings includes processes of:
(1) adding the compound represented by Formula (I) as an IRA to the material and/or to its surroundings,
(2) exposing the material and/or its surroundings to an input energy to form a thermal image; and
(3) measuring a change in a temperature of the material and/or its surroundings by a thermal image detector.

The IRA is preferably added only to the material or only to the surroundings of the material, in order to enhance the contrast between them.

The IRA may be used in a solid form, in a solution or in dispersion liquid, or in vaporized or atomized to form an aerosol, and then may be substantially oriented to a target site or a site for image formation. Such orientation may be performed by a natural affinity of the IRA for a particular target site, or may be performed by a targeted imaging process when a carrier is used, in which antibodies or vectors of other similar systems may be included. The IRA may be directly placed in the target region. Alternatively orientation may be performed, for example, by restricting the mobility of the IRA so that the IRA remains substantially in the target region by controlling the inherent solubility, pH or lipid/water partition of the IRA.

Examples of the material include any part of a human or animal body, a plant or other vegetation, a building or industrial construction, and a motor vehicle and applications or supports in aviation transport, such as paper including, for example, rag paper, printer quality paper, currency grade paper, plastics-coated or laminated paper or other supports generally used for documents or packaging.

As the input energy, a radiation supply source of electronic energy, specifically an infrared ray, is used, and a wavelength of from 750 nm to 1500 nm light is preferable, and the electromagnetic spectrum of near infrared rays of from 800 nm to 1000 nm is particularly preferable. Examples of a typical radiation supply source include a simple halogen bulb having an emission spectrum with a substantial portion in the near infrared region, a light emitting diode (LED) and an IR semiconductor laser such a GaAlAs laser (emission at 785 nm).
Irradiation time may be selected appropriately to give an optimum image without direct heating which interferes with the strength of the signal.

The thermal image detector may be any device that detects and preferably records a thermal energy difference between or within the inside of a material and/or its surroundings such as a thermal imaging camera (for example, Therma CAM (registered trademark) SC1000 camera, manufactured by FLIR Systems, Boston, USA). The thermal imaging camera preferably contains a charge couple device (CCD) sensitive to light having a wavelength from 1.5 to 15 microns, more preferably from 3.4 to 5 microns. Image manipulation and data handling are performed using appropriate computer software such as Thermagram (registered trademark) PRO95 software manufactured by Thermoteknix Systems Limited, Cambridge, UK or the like.

The IRA can be preferably added to the material and/or its surroundings in the form of a composition containing the IRA and a liquid medium. The IRA can be dissolved or dispersed in a liquid medium.
Examples of the liquid media include water, a mixture of water and an organic solvent, and an organic solvent free from water. It is preferred that the organic solvent contained in the mixture of the water and the organic solvent is a water-miscible organic solvent or a mixture of such a solvent. Examples of the preferred water-miscible organic solvent include alcohols, more specifically methanol and ethanol; dimethylsulfoxide; cyclic amides, particularly 2-pyrrolidone, N-methyl-pyrrolidone and N-ethyl-pyrrolidone; diols, particularly 1,5-pentane-diol, ethylene glycol, thio diglycol, diethylene glycol and triethylene glycol; and mono-C₁₋₄-alkyl and C₁₋₄-alkyl-ethers of diols, more preferably mono-C₁₋₄-alkyl ethers of diols having 2 to 12 carbon atoms, particularly 2-methoxy-2-etboxy-2-ethoxyethanol.

The image to be detected can be formed by applying an ink to the product, particularly, a UV curable ink containing the compound represented by Formula (I) (IRA).

### ii. UV Curable Ink

Examples of the UV-curable ink include an ink containing an alkoxylated or polyalkoxylated acrylate monomer, a photoinitiator and a colorant, as described in U.S. Patent No. 6,114,406. A preferred UV-curable ink may be an UV-curable ink jet composition, and the composition may contain 80% by mass to 95% by mass of a polyfunctional alkoxylated and/or polyalkoxylated acrylate monomer with respect to the total composition, IRA, and a colorant, if desired.

The amount of the acrylate monomer, photoinitiator, IRA and colorant may be varied in accordance with a specific equipment or application. The amount of the photoinitiator is preferably from 1% by mass to 15% by mass with respect to the total composition.
The scope of the polyfunctional alkoxylated or polyalkoxylated acrylate monomer may include at least one di- or tri-acrylate, or an alkoxylated or polyalkoxylated acrylic monomer with higher functionality may be used alone or together with the at least one bi- and/or trifunctional material. The number of alkyleneoxy groups is preferably from 1 to 20 per molecule of the monomer, and each group is preferably C₂₋₄-alkyleneoxy and particularly preferably ethyleneoxy (EO) or propyleneoxy (PO).

Examples of the polyfunctional alkoxylated or polyalkoxylated acrylate include an alkoxylated adduct, preferably an ethoxylated or propoxylated adduct, of neapentylglycol diacrylate, butanediol diacrylate, trimethylpropane tri-acrylate and glyceryl triacrylate.

The ink may also contain 10% by mass or less of a monofunctional alkoxylated or polyalkoxylated acrylate monomer, such as an alkoxylated adduct, particularly an ethoxylated or propoxylated adduct, of at least one tetrahydrofurfuryl acrylate, cyclohexyl acrylate, alkyl acrylate, nonyl-phenol acrylate, and polyethylene- or polypropylene-glycol acrylate.

The ink may also contain 5% by mass or less of a non-alkoxylated, mono- or poly-functional radiation curable monomer, such as octyl acrylate, decyl acrylate, N-vinyl-pyrrolidone, ethyl diglycol acrylate, isobornyl acrylate, ethyl-hexyl acrylate, lauryl acrylate, butanediol monoacrylate, P-carboxyethyl acrylate, i-butyl acrylate, polypropylene glycol monomethacrylate, 2-hydroxyethyl methacrylate, hexanediol di(meth)acrylate, tetraethylene glycol diacrylate, tripropylene glycol diacrylate, butanediol diacrylate, polyethylene glycol diacrylates and triethylene glycol dimethacrylate.

Examples of the commercially available photoinitiator include xanthones, thioxanthones, benzophenones, quinones and phosphine oxides. A co-initiator may be mixed with a primary photoinitiator. Examples of the co-initiator include amines and aminobenzoates. When the ink contains the primary initiator and the co-initiator, the total amount is preferably within a preferred range of the amount of the photo initiator described above. It is preferable that an aminobenzoate and an acrylated amine co-initiator are each used with the xanthone and/or thioxanthone primary photoinitiator.

The term "radiation curable" means that a composition is curable by application of UV irradiation. Such a composition is a substantially colorless-curable varnish or base, or when the compound contains a colorant (that is, a material which provides a visual or related optical property, such as fluorescence), the composition may be an ink. When the colorant is contained, the ink preferably contains from 1% by mass to 10% by mass of the colorant with respect to the total mass.

The colorant is classified into two kinds, that is, (a) a dye which is substantially soluble in the ink composition and (b) a pigment which is dispersed in the ink composition in the form of fine particles by using a suitable dispersant. Examples of a typical pigment include Pigment Red 57:1, Pigment Red 52:2, Pigment Red 48:2, Pigment Blue 15:3, Pigment Green 7, Pigment Yellow 83, Pigment Yellow 13 and Pigment White 6. When the colorant is carbon black or contains carbon black, it is not usually necessary to add an IRA because carbon black absorbs the IR region of the spectrum strongly.

The ink may also contain other minor components such as a surface additive, a levelling additive, a photoinitiator stabilizer, a wetting agent and a pigment stabilizer. For example, the latter may be particularly a form of high molecular weight block co-polyiners such as a polyester, polyurethane or polyacrylate type, and are usually contained at about from 2.5% by mass to 100% by mass with respect to the pigment. Specific examples include Disperbyk 161 or 162 (manufactured by BYK Chemie) and Solsperse hyperdispersans (manufactured by Avecia). Examples of the photoinitiator stabilizer include such as those described in European Patent (EP-A) No. 0465039. Examples of the surface additive include a non-ionic surfactant such as Fluorad FC430 (manufactured by 3M Corp.). The amount of the surface additive (when the surfactant is contained) is preferably in a range of from 0.1 % by mass to 10% by mass with respect to the total mass of the ink.

The ink or varnish is preferably substantially or totally free of an organic solvent. Thus, the amount of the organic solvent is preferably less than 10% by mass, more preferably less than 5% by mass, especially preferably less than 1% by mass and particularly preferably less than 0.1% by mass, with respect to the total mass.

The compound represented by Formula (I) is not limited to use only in a UV curable ink, but may be used in any other appropriate UV curable ink which is soluble or dispersible.

### iii. Security System

### (a) Ink for Lithographic Printing

The ink for lithographic printing may be an ink in which at least the compound represented by Formula (I) and a cross-linked resin prepared by, for example, grafting a polyepoxide onto a carboxyl group of a phenolic- resin or a maleic acid-modified rosin ester resin are included and are solubilized with an aliphatic alcohol having at least 12 carbon atoms.

The phenolic- or maleic acid-induced rosin ester resin preferably has a number average molecular weight of from about 1,500 to 3,000. The polyepoxide is preferably diepoxide, more preferably aromatic or alicyclic diepoxide, and especially preferably bisphenol A diepoxide. The molecular weight of the polyepoxide is preferably 560 daltons or less, more preferably from 100 to 500 daltons, and especially from 300 to 500 daltons.

The phenolic resin or maleic acid-induced rosin ester resin is preferably a reaction product of four components, which are (a) polyol, (b) monobasic aliphatic carboxylic acid, (c) rosin or modified rosin, and (d) polycarboxylic acid and/or anhydride thereof. The polyol is preferably thiol and examples thereof include trimethylolethane, trimethylolpropane, glycerol and hexanetriol. The preferred monobasic aliphatic carboxylic acid has about 8 to 20 carbon atoms, and examples thereof include stearic acid, lauric acid, palmitic acid, oleic acid and refined tall oil fatty acid. The preferred rosin or modified rosin is selected from tall oil rosin, wood rosin, hydrogenated rosin and dehydrogenated rosin. The polycarboxylic acid or anhydride thereof may be aliphatic or aromatic and examples thereof include phthalic anhydride, trimellitic anhydride, tetrahydrophthalic anhydride, maleic anhydride, isophthalic acid, fumaric acid, and mixtures thereof.

The phenolic resin or maleic acid-modified rosin resin may be prepared by a two-step process. In the first step, the polyol, the monobasic aliphatic carboxylic acid, and the rosin or modified rosin are reacted at a temperature of from about 250°C to 290°C and preferably from about 260°C to 280°C, whereby an acid number is from about 1 to 10. In the second step, the polycarboxylic acid or anhydride is added thereto and the reaction is continued at a temperature of from about 150°C to 220°C, and preferably from about 170°C to 200°C, whereby an acid number is from about 20 to 90, and preferably from about 20 and 50. As a result, all of the monobasic aliphatic carboxylic acid groups and most of the rosin carboxylic acid groups are reacted at about 250°C to 290°C and the aromatic carboxylic acid as a pendant group is added at about 150°C to 220°C.

Polymer oily matter obtained by grafting the polyepoxide onto the ink resin remains in insoluble, by which squalene (sebum) resistance is improved, thereby tending to release the solvent due to improved heatset drying. Since the cross-linked resin is slightly difficult to be dissolved, the cross-linked resin is kept in a solution by adding an aliphatic alcohol having at least 12 carbon atoms, preferably 12 to 24 carbon atoms, and more preferably 12 to 13 carbon atoms, such as Neodo123 (trade name, manufactured by Shell Oil Co.).

In order to achieve ink-roller stability required for high-speed lithographic web printing, a high boiling-point petroleum-distillate varnish solvent such as Magic 470 and Magie 500 (hydrocarbon solvents, manufactured by Magie Brothers Oil Company, 9101 Fullerton Ave., Franklin Park, Ill.) is also contained. The aliphatic alcohol is used to stabilize the resin in the solvent. When the balance of solubility is affected by evaporation of alcohol in some degree during the heat-drying process and adsorption into the paper, the resin precipitates out of the solution, whereby an ink film dries to touch. The more alcohol evaporates off, the faster the ink dries. The appropriate amount of alcohol is an amount such that the ink moves through the printing rollers of the press to print on the paper without drying during the printing process.

The compound represented by Formula (I) (IRA) is preferably from about 0.01% by mass to 5.0% by mass and preferably from about 0.1% by mass to 3.0% by mass, with respect to the total mass of the ink.
The IRA can form an infrared absorbing ink that is detectable, after application to a product or a support, only when the product, the support, or the like is irradiated with an infrared ray by an infrared detector.

When the ink contains a colorant, the printed pattern can be detected visually. The printed support is distinguishable from a support printed with an ink that does not contain the IRA, in a similar manner. As the colorant, one of a variety of conventional organic or inorganic pigment may be used, and example thereof include molybdate orange, titanium white, phthalocyanine blue, and carbon black. The amount of colorant is preferably from about 5% by mass to 30% by mass with respect to the total mass of the ink.

The ink also may contain a modifier such as a plasticizer; a wetting agent for the colorant; a levelling agent such as lanolin, paraffin wax, and natural wax; and a slip agent such as low molecular weight polyethylenes and microcrystalline petroleum wax. The modifier can be used in a varying amount within the range of about 3% by mass or less, and preferably about 1% by mass or less, with respect to the total mass of the ink. Other components may be conventionally used in the ink and the coatings to modify adhesion and toughness, and other basic properties may also be used.

The lithographic printing ink may be prepared by a mixing and filter process, or the like, using an appropriate method, such as a three-roll mill or the like, in accordance with a known dispersion method. The ink can be applied to the support, preferably paper, by an appropriate known and conventional method.

### (b) Solvent Based Ink for Ink-Jet Recording (IJP)

The solvent-based ink for ink jet recording is an ink in which at least the compound represented by Formula (I) and at least one aromatic sulfonamide or hydroxybenzoic acid ester are contained and are dissolved in an organic solvent.

The aromatic sulfonamide is preferably toluenesulfonamide substituted as necessary such as p-toluenesulfonamide, N-ethyl-p-toluenesulfonamide, N-butyl-p-toluene-sutfonamide and N-cyclohexyl-p-toluenesulfonamide. The aromatic sulfonamide is preferably contained in the organic solvent-based IJP ink in an amount of from 0.1% by mass to 40% by mass.

The hydroxybenzoic acid ester is preferably an alkyl ester, particularly those containing from 6 to 12 carbon atoms, such as 2-ethylhexyl p-hydroxybenzoate and n-nonyl p-hydroxy-benzoate. The hydroxybenzoic acid ester is preferably contained in the organic solvent-based IJP ink in an amount of from 0.1% by mass to 40% by mass.

The aromatic sulfonamide and hydroxybenzoic acid ester have a high polarity, therefore crystallization of the dye is inhibited very effectively.
The compound represented by Formula (I) (IRA) is preferably from about 0.01% by mass to 5.0% by mass, and more preferably from about 0.1% by mass to 3.0% by mass, with respect to the total mass of the ink.
The IRA can form an infrared absorbing ink that is detectable, after application to goods or a support, only when the goods, the support, or the like is irradiated with an infrared ray by an infrared detector.

When the ink contains a colorant, the printed pattern can be detected visually. The printed support is distinguishable from a support printed with an ink that does not contain the IRA, in a similar manner. The colorant may be an appropriate dye that is soluble in an organic solvent, or may be the aromatic sulfonamide or hydroxybenzoic acid ester. Generally, examples of the dye for use include an azo dye, a metal complex salt dye, a naphthol dye, an anthraquinone dye, an indigo dye, a carbonium dye, a quinoimine dye, a cyanine dye, a quinoline dye, a nitro dye, a nitroso dye, a benzoquinone dye, a naphthoquinone dye, a naphthalimide dye, a perinone dye, and a phthalocyanine dye. These dyes may be used singly or in combination of two or more thereof.
The content of the dye is preferably in a range of from 0.1% by mass to 10% by mass and more preferably from 0.5% by mass to 5% by mass, with respect to the total mass of the ink.

The type of the organic solvent used in the IJP ink depends on a specific dye or dye mixture in some degree. However, as most of dyes are polar, a high polar solvent functions as a good solvent and a less polar solvent functions as a poor solvent. Therefore, a high polar solvent having a high ability to dissolve a dye is preferred for preparing the IJP ink. However, the less polar solvent can be used when mixed with an aromatic sulfonamide or hydroxybenzoic acid ester or is combined with a solvent having a higher polarity. Specific examples of the organic solvent that can be used for the IJP ink include: aliphatic hydrocarbons; naphthenic hydrocarbons; aromatic hydrocarbons such as mono- or di-substituted alkylnaphthalenes, an alkyl derivative of biphenyls, xylylethane or phenethylcunnene; glycols; mono- or di-alkyl ethers of glycols and esters of glycols; fatty acids and esters thereof; a nitrogen-containing compound such as an amide and pyrrolidone compound. The organic solvent that can be used for preparing the IJP inks is not limited thereto.

As the solvent, a solvent having a higher the boiling point is more preferable, from the viewpoint of less evaporation and drying speed. However, when a solvent having a high boiling point is used, the ink has a tendency to be viscous and to be difficult to eject the solvent smoothly. In contrast, a solvent having a lower boiling point has a tendency to produce an ink that dries too fast at a nozzle orifice. Therefore, a solvent having a desired viscosity and a boiling point should be selected in consideration of the measure adopted by a print head to prevent ink drying.

### (c) Gravure/Intaglio Printing Ink

A gravure ink for intaglio printing can contain the compound represented by Formula (I), a resin, a volatile solvent and a finely pulverized pigment (preferably dispersed in an ink vehicle that formed from an antioxidant or antioxidant composition).

The resin for the gravure ink depends on the solvent, the support to be printed and the end use of the printed matter. Examples of the resin suitable for manufacturing the ink including a gravure type are described in "Synthetic Resins" written by Werner Husen, The American Ink Maker, June 1952, page 63, and "Synthetic Resins for Inks," written by John P. Petrone, The American Ink Maker, Vol. 49, March-October, 1971. Examples of the resin for use include rosin and modified rosins, such as a calcium and zinc resinate and a variant thereof. Examples of other suitable resins include:
· a various modified product of a petroleum resin or a cyclopentadiene resin These examples are described in U.S. Patatent No. 3,084,147 and U.K. Patent No. 1,369,370, and
a modified resin having a softening point of 145°C and having a stable viscosity and the ability capable of inducing excellent printability, as described in Japanese Patent No 47994/72 (the modified resin can be prepared by collecting a fraction having a boiling point at from 140°C to 220°C from a cracked oil which is obtained by pyrolyzing petroleum; polymerizing the fraction using a Friedel-Craft catalyst to obtain a resin having a softening point of 160°C; reacting the resin with unsaturated carboxylic acid or anhydride thereof at an amount of from 0.01 mole to 0.4 mole per 100 gram of the resin; and then esterifying the resulting resin using a monovalent alcohol in an amount of from 0.2 moles to 2.0 moles per 1 mole of the unsaturated carboxylic acid or anhydride).

The volatile solvent may be an aliphatic or an alicyclic hydrocarbon such as hexane, heptane and cyclohexane, or an aromatic hydrocarbon such as xylene, toluene (for example, tolusol 25), high flash naphtha, benzene and chlorobenzene. Examples of the solvent other than above include alkanols having 1. to 4 carbon atoms, acetate of alkanols having 1 to 5 carbon atoms, glycol ethers having a boiling point of from 115°C to 180°C, aliphatic ketones having 1 to 5 carbon atoms and cyclohexanone. The resin should be soluble in the solvent and readily separated therefrom. Since the drying of the gravure ink is caused by evaporation of the solvent, the ink vehicle is substantially a resin and a solvent. Depending upon a specific combination of the resin and the solvent, various types of vehicles can be used.

The preferred antioxidant is a phenolic antioxidant or an amine antioxidant, and the preferred antioxidant composition is a mixture of a phenolic antioxidant and an amine antioxidant. The preferred antioxidant composition contains from about 10% by mass to 90% by mass, more preferably from about 25% by mass to 75% by mass, of the phenolic antioxidant; and from about 90% by mass to 10% by mass, more preferably from about 75% by mass to 25% by mass, of the amine antioxidant.

Examples of the amine antioxidant include octylated diphenylamine, isopropoxy diphenylamine, an aldol-α-naphthylamine condensation product of diphenylamine and acetone, N,N'-diphenyl-p-phenylenediamine, phenyl-β-naphthylamine, polymerized 1,2-dihydro-2,2,4-trimethylquinoline, N,N'-di(2-octyl)-p-phenylenediamine, other aromatic amines, diphenylamines, and a mixture thereof. Examples of the phenolic antioxidant include 4,4'-isopropylidene-diphenol, styrenated phenol, hindered phenol, 4,4'-thiobis (6-t-butyl-o-cresol), p-butylphenol, p-(i-pz-opyl)phenol, 2,4-dimethyl-6-octylphenol, 2,6-di-t-butyl-4-methylphenol, 2,6-di-t-butyl-4-ethylphenol, 2,6-di-t-butyl-4-n-butylphenol, 2,2'-methylenebis-(4-methyl-6-t-butylphenol), 2,2'-methylenebis(4-ethyl-6-t-butyl-phenol), 2,4-dimethyl-6-t-butylphenol, 4-hydroxymethyl-2,6-di-t-butylphenol, n-octadecyl-beta (3,5-di-t-butyl-4-hydroxyphenyl) propionate, and a mixture thereof. A preferred phenolic antioxidant is sterically mixed phenols.

The gravure ink for intaglio printing, including the antioxidant or antioxidant composition, may be prepared by a common method. For example, 100 parts by mass of the resin and 1 part by mass of the antioxidant or antioxidant composition are dissolved in 200 parts by mass or less of a high boiling point petroleum solvent, thereby preparing the ink vehicle. A mixture of a preferred solvent such as 70% by mass of toluene and 4% by mass of xylene and 26% by mass of lactol spirit has a Kauri butanol value of about 105 (in contrast to an aliphatic solvent with a Kauri butanol value of from 35 to 45).

By containing the compound represented by Formula (I) (IRA) in the ink vehicle as a dispersion liquid, in a a range of from about 0.01 % by mass to 5.0% by mass, and preferably from about 0.1 % by mass to 3.0% by mass, with respect to the total mass of the ink, a transparent infrared absorbing ink in which the support can be detected, after application to a support, only when the support is irradiated with an infrared ray by an infrared sensitive detector can by formed. When the ink contains a colorant, the printed pattern can be detected visually.
When the ink contains a colorant, the printed pattern can be detectable visually. The printed support (a material printed) can also be distinguished from a support printed with an ink that does not contain the IRA, in a similar manner.

The IRA or colorant (such as Phthalocyanine Blue, Benzidine Yellow, channel black, Carmine 6B or titanium white) is added to the ink vehicle, preferably as a dispersion liquid, and the mixture is placed in a ball mill and ground until a dispersion liquid in which the pigment is present uniformly in the ink vehicle is obtained. The obtained ink concentrate can be adjusted to a concentration appropriate for use in printing by diluting with an additional solvent.

A general gravure ink composition for intaglio printing contains from about 0.005% by mass to 0.5% by mass of the antioxidant composition, from about 10% by mass to 50% by mass of the resin and from about 0.01% by mass to 5.0% by mass (preferably, from 0.1 by mass to 3.0% by mass) of the compound represented by Formula (I) (IRA), with respect to the total mass of the ink, and/or contains from about 50 parts by mass to 100 parts by mass of colorant per 100 parts by mass of the resin, and the remainder is formed substantially from a mixture of hydrocarbon solvents such as toluene, xylene and a lactol distillate. The viscosity of the ink at the point of use is preferably 0.5 Pa·s (5 P) or less, and more preferably from 0.05 Pa·s to 0.1 Pa·s (from 0.5 to 1.0 P). The amount of antioxidant composition used is preferably from 0.005% by mass to 0.5% by mass and more preferably from 0.025% by mass to 0.5% by mass with respect to the total mass of the ink.

Other additives may be added in order to improve printability, flow behavior and pigment wetting, preferably in an amount of from 1% by mass to 15% by mass (more preferably from 1% by mass to 10% by mass), with respect to the resin. Wax, such as ester wax, amide wax, or hydrocarbon wax, may be added in an amount of from 0.1% by mass to 5% by mass.

Other compatible additives such as ethyl cellulose or ethyl hydroxy cellulose may be used to improve ink adhesion to a film, scuff resistance, gloss, and the like. The ink for printing is preferably used without a plasticizer; however, the plasticizer can be added to achieve special effects.

Examples of the support include those commonly used in intaglio printing such as paper, cellophane and metal films (for example, aluminum film).

The labeled liquid can be detected again, for example by using an appropriate method at the time of use. According to the invention, by using the compound represented by Formula (I), a liquid is labeled and a labeled liquid is identified by detecting the labeled liquid. Accordingly, fuel oil and diesel oil can be distinguished from each other, for example.

Examples of the solvent that can be labeled by the compound represented by Formula (I) include, particularly an organic solvent such as alcohols (for example, methanol, ethanol, propanol, isopropanol, butanol, isobutanol, s-butamol, pentanol, isopentanol, neopentanol, and hexanol); glycols (for example, 1,2-ethylene glycol, 1,2- or 1,3-propylene glycol, 1,2-, 2,3- or 1,4-butylene glycol, di- or tri-ethylene glycol, and di- or tri-propylene glycol); ethers (for example, methyl-t-butyl ether, 1,2-ethylene glycol monomethyl or dimethyl ether, 1,2-ethylene glycol monoethyl or diethyl ether, 3-methoxypropanol, 3-isopropoxypropanol, tetrahydrofuran and dioxane); ketones (for example, acetone, methylethyl ketone and diacetone alcohol); esters (for example, methyl acetate, ethyl acetate, propyl acetate, and butyl acetate); aliphatic or aromatic hydrocarbons (for example, pentane, hexane, heptane, octane, iso-octane, petroleum ether, toluene, xylene, ethyl benzene, tetralin, decalin, dimethylnaphtalene, and mineral spirit); mineral oil (for example, gasoline, kerosene, diesel oil and fuel oil); natural oil (for example, olive oil, soy bean oil, and sun flower oil); or natural or synthetic engine oil, and pressure medium oil or gear oil (for example, engine oil, sewing-machine oil, and brake oil, for an automobile).

By using the compound represented by Formula (I), a heat ray-shielding material can be produced, for example, under the condition described in JP-A No. 2000-17184. By containing the compound represented by Formula (I), heat resistance and wet heat resistance are excellent and a long term usage can be achieved.

In the present application, JP-A No. 2009-44510 which has been filed in Japan on February 26, 2009 is included as a reference, and the entire disclosure of which is incorporated herein by reference.

### EXAMPLES

The invention is further explained in detail by the following Examples. However, the invention is not limited by the Examples as long as the gist is maintained. Further, unless specifically stated otherwise, "%" is on a mass basis.

### (Example 1)

### 1. Production of ink compositions (1a) to (I g) for lithographic printing

Ink compositions for lithographic printing were prepared by mixing each component according to the composition shown in Table 1 below. The ink compositions can be used for security printing ink.

### 2. Performance evaluation

A sample was prepared by coating, on paper, each of the ink compositions for lithographic printing thus obtained, and the following evaluations were performed.

### 1) Evaluation of light resistance

Irradiation by a xenon lamp at 95,000 lux was performed for the time shown in Table 2 below. Light resistance was evaluated for each sample by determining a residual ratio by measuring the concentration of each infrared-absorbing compound at a spectroscopic absorption maximum wavelength before and after the irradiation (measurement in a wavelength region of from 200 nm to 1600 nm).

### 2) Evaluation of heat fastness and wet heat fastness

Each of the same samples as above was left to stand under the conditions of a temperature of 80°C and under the condition of 60°C and relative humidity (RH) of 90% for the time shown in Table 2 below. Heat fastness and wet heat fastness were evaluated for each sample by determining a residual ratio by measuring the concentration of each infrared-absorbing compound at a spectroscopic absorption maximum wavelength before and after being left to stand (measurement in a wavelength region of from 200 nm to 1600 nm).

Further, each ink after the irradiation or being left to stand, under the conditions described above was coated on paper and a plastic sheet, to draw an image, and then detection was performed using a scanner (laser wavelength of 940 nm). As a result, the images formed by the ink compositions of the invention could be detected well. Meanwhile, the detectability of the comparative ink compositions was deteriorated after being left to stand.

**[Table 1]**

| Material | Addition amount (% by mass) |
|---|---|
| Phenolic resin modified with Bisphenol A epoxide | 46.0 |
| C. I. Pigment Blue 15:3 | 30.2 |
| Teflon (registered trademark of E. 1. Du Pont de Nemours & Co., Inc.) | 7.0 |
| Bodied Tung Oil (commercially available Tung oil in general) | 10.0 |
| Co/Mn drying agent (trade name: NAPHTHEX COBALT, manufactured by NIHON KAGAKU SANGYO CO.,LTD.) | 2.0 |
| Exemplary compound (1-4), (1-21), or (1-34) or Comparative compound (shown in Table 2) | 0.6 |
| BHT (antioxidant, dibutyl hydroxytoluene, manufactured by Tokyo Chemical Industry Co., Ltd.) | 2.0 |
| Hydrocarbon solvent (Magie 470) (manufactured by Magie Brothers Oil Co.) | 2.3 |

**[Table 2]**

| Ink No | Compound | Light resistance | Heat fastness | Wet heat fastness | Remarks |
|---|---|---|---|---|---|
| | | (3 hours) | (100 hours) | (100 hours) | |
| 1a | Exemplary compound (I-4) | 82 | 86 | 81 | The invention |
| 1 b | Exemplary compound (I-21) | 76 | 78 | 74 | The invention |
| 1c | Exemplary compound (I-34) | 71 | 80 | 76 | The invention |
| 1d | Exemplary compound (I-52) | 75 | 83 | 78 | The invention |
| 1e | Comparative compound-1 | 50 | 69 | 58 | Comparative example |
| 1f | Comparative compound-2 | 38 | 62 | 40 | Comparative example |
| 1g | Comparative compound-3 | 41 | 83 | 70 | Comparative example |

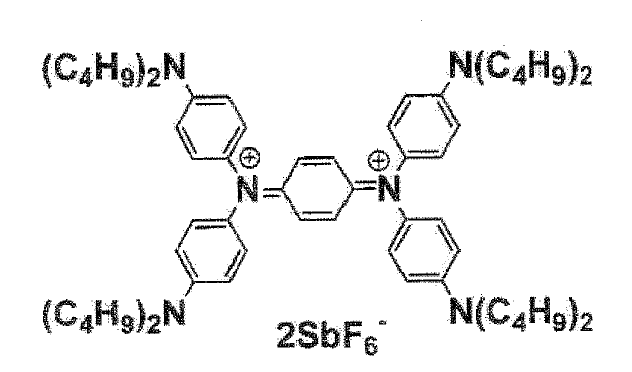

### Comparative compound-1

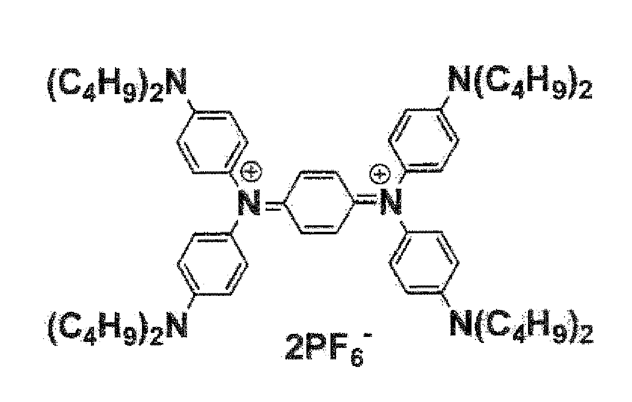

### Comparative compound-2

V=O-3,6-(4-methylphenylthio)₈phthalocyanine

### Comparative compound-3

From the above, it was found that the compound represented by Formula (I) has better fastness than the Comparative compounds having a similar skeleton. In particular, the compound represented by Formula (1) exhibited more excellent light fastness compared to the compound described in JP-T No. 2005-537,319 (Comparative compound-3).

### (Example 2)

### 1. Production of ink compositions for intaglio printing

An ink composition for intaglio printing was prepared by mixing each component according to the composition shown in Table 3 below. The ink composition can be used for security printing ink.

### 2. Performance evaluation

Next, a sample was prepared by coating, on paper, each of the ink compositions for intaglio printing, and the following evaluations were carried out.

### 1) Evaluation of light resistance

Each of the samples thus obtained was irradiated by a xenon lamp at 95,000 lux for the time shown in the following Table 4. Light resistance was evaluated for each sample by determining a residual ratio by measuring the concentration of each infrared-absorbing compound at a spectroscopic absorption maximum wavelength before and after the irradiation (measurement in a wavelength region of from 200 nm to 1600 nm)

### 2) Evaluation of heat fastness and wet heat fastness

Each of the same samples as above was left to stand under the conditions of a temperature of 80°C and under the condition of 60°C and relative humidity (RH) of 90% for time shown in Table 4 below. Heat fastness and wet heat fastness were evaluated for each compound by determining a residual ratio by measuring the concentration of each infrared-absorbing compound at a spectroscopic absorption maximum wavelength before and after being left to stand (measurement in a wavelength region of from 200 nm to 1600 nm).

Further, each ink after the irradiation or being left to stand, under the conditions described above was coated on paper and a plastic sheet, to draw an image, and then detection was performed using a scanner (laser wavelength of 940 nm). As a result, the images formed by the ink compositions of the invention could be detected well. Meanwhile, the detectability of the comparative ink compositions was deteriorated after being left, to stand.

**[Table 3]**

| Material | Addition amount (% by mass) |
|---|---|
| Resin: curable rosin oil | 50.0 |
| Anti-oxidant (trade name: HALS, manufactured by Ciba Specialty Chemicals) | 0.4 |
| Toluene/xylene/lactol solvent (mixing ratio: 1/1/1) | 25.0 |
| C.I. Pigment Blue 15:3 | 24.0 |
| Exemplary compound (I-4), (I-23), or (I-31) or Comparative compound (shown in Table 4) | 0.6 |

**[Table 4]**

| Ink No | Compound | Light resistance | Heat fastness | Wet heat fastness | Remarks |
|---|---|---|---|---|---|
| | | (3 hours) | (100 hours) | (100 hours) | |
| 2a | Exemplary compound (I-4) | 78 | 82 | 77 | The invention |
| 2b | Exemplary compound (1-23) | 70 | 74 | 62 | The invention |
| 2c | Exemplary compound (I-31) | 76 | 70 | 68 | The invention |
| 2d | Exemplary compound (I-53) | 72 | 70 | 69 | The invention |
| 2e | Comparative compound-1 | 47 | 60 | 42 | Comparative example |
| 2f | Comparative compound-2 | 30 | 45 | 33 | Comparative example |
| 2g | Comparative compound-3 | 38 | 70 | 53 | Comparative example |

From the above, it was found that the compound represented by Formula (I) has better fastness than the Comparative compounds having a similar skeleton. In particular, the compound represented by Formula (I) exhibited more excellent light fastness compared to the compound described in JP-TNo. 2005-537,319 (Comparative compound-3).

### (Example 3)

### 1. Production of UV-curable ink compositions

A UV-curable ink composition was prepared by mixing each component according to the composition shown in Table 5 below. The ink composition can be used for security printing ink.

### 2. Performance evaluation

Next, a sample was prepared by coating, on paper, each UV-curable ink composition, and the following evaluations were carried out.

### 1) Evaluation of light resistance

Each of the samples thus obtained was irradiated by a xenon lamp at 95,000 lux for the time shown in the following Table 6. Light resistance was evaluated for each compound by determining a residual ratio by measuring the concentration of each infrared-absorbing compound at a spectroscopic absorption maximum wavelength before and after the irradiation (measurement in a wavelength region of from 200 nm to 1600 nm).

### 2) Evaluation of heat fastness and wet heat fastness

Each of the same samples as above was left to stand under the conditions of a temperature of 80°C and under the condition of 60°C and relative humidity (RH) of 90% for the time shown in Table 6 below. Heat fastness and wet heat fastness were evaluated for each compound by determining a residual ratio by measuring the concentration of each infrared-absorbing compound at a spectroscopic absorption maximum wavelength before and after being left to stand (measurement in a wavelength region of from 200 nm to 1600 nm).

Further, each ink after the irradiation or being left to stand, under the conditions described above was coated on paper and a plastic sheet, to draw an image, and then detection was performed using a scanner (laser wavelength of 940 nm). As a result, the images formed by the ink compositions of the invention could be detected well. Meanwhile, the detectability of the comparative ink compositions was deteriorated after being left to stand.

**[Table 5]**

| Material | Addition amount (% by mass) |
|---|---|
| Isobornylacrylate | 50.0 |
| Ethoxylated trimethylol propane triacrylate | 27.0 |
| Propoxylated neopentyl glycol diacrylate | 16.0 |
| C.I. Pigment blue 15:3 | 2.0 |
| Exemplary compound (I-4), (I-21), (I-24), or (1-32), or Comparative compound (shown in Table 6) | 0.5 |
| High molecular weight dispersant: polyacrylic acid partial alkyl ester | 0.5 |
| 2-Ethylanthraquinone (photoinitiator) | 3.7 |
| Fluorocarbon surface additive (trade name: EF-351, manufactured by F-top Co., Ltd.) (hydrocarbon acrylate-perfluorocarbon acrylate copolymer) | 0.3 |

**[Table 6]**

| Ink No. | Compound | Light resistance | Heat fastness | Wet heat fastness | Remarks |
|---|---|---|---|---|---|
| | | (1 hour) | (50 hours) | (50 hours) | |
| 3a | Compound (1-4) of Formula (I) | 85 | 89 | 80 | The invention |
| 3b | Compound (I-21) of Formula (I) | 77 | 74 | 74 | The invention |
| 3c | Compound (1-24) of Formula (I) | 69 | 72 | 76 | The invention |
| 3d | Compound (I-32) of Formula (I) | 82 | 83 | 78 | The invention |
| 3e | Compound (1-52) of Formula (1) | 74 | 77 | 77 | The invention |
| 3f | Comparative compound-1 | 62 | 71 | 51 | Comparative example |
| 3g | Comparative compound-2 | 51 | 67 | 42 | Comparative example |
| 3h | Comparative compound-3 | 46 | 80 | 73 | Comparative example |

From the above, it was found that the compound represented by Formula (I) has better fastness than the Comparative compound having a similar skeleton. In particular, the compound represented by Formula (I) exhibited more excellent light fastness compared to the compound described in JP-T No. 2005-537,319 (Comparative compound-3).

### (Example 4)

### 1. Production of solvent-based ink compositions

A solvent-based ink composition was prepared by mixing each component according to the composition shown in Table 7 below. The ink composition can be used for security printing ink.

### 2. Performance evaluation

Next, a sample was prepared by coating, on paper, each ink composition, and the following evaluations were carried out.

### 1) Evaluation of light resistance

Each of the samples thus obtained was irradiated by a xenon lamp at 95,000 lux for the time shown in 8 below. Light resistance was evaluated for each compound by determining a residual ratio by measuring the concentration of each infrared-absorbing compound at a spectroscopic absorption maximum wavelength before and after the irradiation (measurement in a wavelength region of from 200 nm to 1600 nm).

### 2) Evaluation of heat fastness and wet heat fastness

Each of the same samples as above was left to stand under the conditions of a temperature of 80°C and under the condition of 60°C and relative humidity (RH) of 90% for the time shown in Table 8 below. Heat fastness and wet heat fastness were evaluated for each compound by determining a residual ratio by measuring the concentration of each infrared-absorbing compound at a spectroscopic absorption maximum wavelength before and after being left to stand (measurement in a wavelength region of from 200 nm to 1600 nm).

Further, each ink after the irradiation or being left to stand under the conditions described above was coated on paper and a plastic sheet, to draw an image, and then detection was performed using a scanner (laser wavelength of 940 nm). As a result, the images formed by the ink compositions of the invention could be detected well. Meanwhile, the detectability of the comparative ink compositions was deteriorated after being left to stand.

**[Table 7]**

| Material | Addition amount (% by mass) |
|---|---|
| Oil-soluble copper phthalocyanine: Pigment Blue 15 | 3.4 |
| Phenethylcumene | 80.0 |
| Exemplary compound (I-4), (I-31), or (I-32) or Comparative compound (shown in Table 8) | 0.6 |
| Diethylene glycol hexyl ether | 14.0 |
| N-Butyl-p-toluene sulfonamide | 1.0 |

**[Table 8]**

| Ink No. | Compound | Light resistance | Heat fastness | Wet heat fastness | Remarks |
|---|---|---|---|---|---|
| | | (1 hour) | (50 hours) | (50 hours) | |
| 4a | Compound (I-4) of Formula (I) | 86 | 88 | 81 | The invention |
| 4b | Compound (I-31) of Formula (I) | 84 | 78 | 74 | The invention |
| 4c | Compound (I-32) of Formula (I) | 78 | 81 | 70 | The invention |
| 4d | Comparative compound-1 | 57 | 62 | 48 | Comparative example |
| 4e | Comparative compound-2 | 50 | 51 | 31 | Comparative example |
| 4f | Comparative compound-3 | 44 | 71 | 64 | Comparative example |

From the above, it was found that the compound represented by Formula (I) has better fastness than the Comparative compound having a similar skeleton, In particular, the compound represented by Formula (1) exhibited more excellent light fastness compared to the compound described in JP-T'No. 2005-537,319 (Comparative compound-3).

Although an ink, a toner, and detection of an image are mainly explained in the above examples, signal conversion or proving the authenticity of goods or a support can also be achieved in a similar manner as described above. Similar results can also be obtained when a method of producing mineral oil or a heat ray-shielding material, a polymer welding method, and a method of producing a lithographic printing plate are performed.

Hereinafter, specific examples of the synthesis of compounds used in the invention are shown.

### (Synthetic example 1)

### Synthesis of Exemplary compound (I-4) is shown.

Exemplary compound (I-4) was synthesized based on the following reaction scheme.

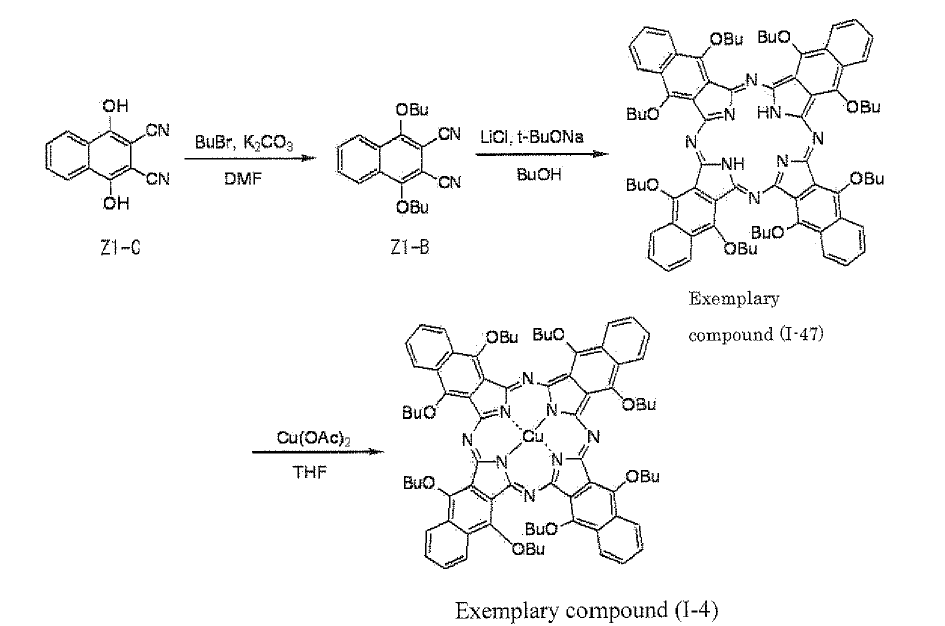

126.1 g of2,3-dicyano-1,4-dihydronaphthoquinone (Z1-C), 205.6 g of butyl bromide, 207.3 g of potassium carbonate, and 350 mL of dimethyl formamide (350 mL) were added to a 1 L three neck flask, and stirred for 4 hours at an inside temperature of 90°C. After cooling to an inside temperature of 50°C and then adding 300 mL of water dropwise over 1 hour, the mixture was cooled to 10°C over 2 hours. After the mixture was stirred at 10°C for 1 hour, the obtained slurry was filtered and washed with 300 mL of methanol and 300 mL of water, thereby obtaining 174.1 g of Compound (Z1-B) (yield of 90%).

64.5 g of Exemplary compound (Z1-B), 50.9 g of lithium chloride, 48.1 g of sodium t-butoxide, and 300 mL of butanol were added to a 2 L three neck flask, and heating was performed for 4 hours at 90°C. After the mixture was cooled to 50°C, 400 mL of water, 30 mL of acetic acid, and 1 L of chloroform were added thereto to extract an organic layer. Subsequently, rinsing was carried out two times using 400 mL of water, and the organic layer thus obtained was dried with anhydrous magnesium sulfate and concentrated to 250 g with a rotary evaporator. To the condensate, 400 mL of hexane was added at an inside temperature of 50°C, and then cooling was performed to -10°C over 2 hours while stirring. Then, the resultant was left to stand at -10°C for 12 hours, and a produced crystal was filtered under a reduced pressure and washed with 300 mL of methanol, thereby obtaining 46.4 g of Exemplary compound (I-47) (yield of 72%). When the obtained compound was measured with an MS spectrum, a peak corresponding to the molecular weight was obtained.

After 10 g of Compound (I-47) was dissolved in 1 L of THF, 1.78 g of copper acetate (1.5 equivalent amounts) was added thereto, and then heating was performed at 60°C for 4 hours. After the reaction solution was cooled, water was added thereto, and the organic layer was washed twice and concentrated, thereby obtaining 10. g of Compound (I-4) (yield of 97%).

### (Synthetic example 2)

### Synthesis of Exemplary compound (I-47) is shown.

Exemplary compound (1-47) was synthesized based on the following reaction scheme.

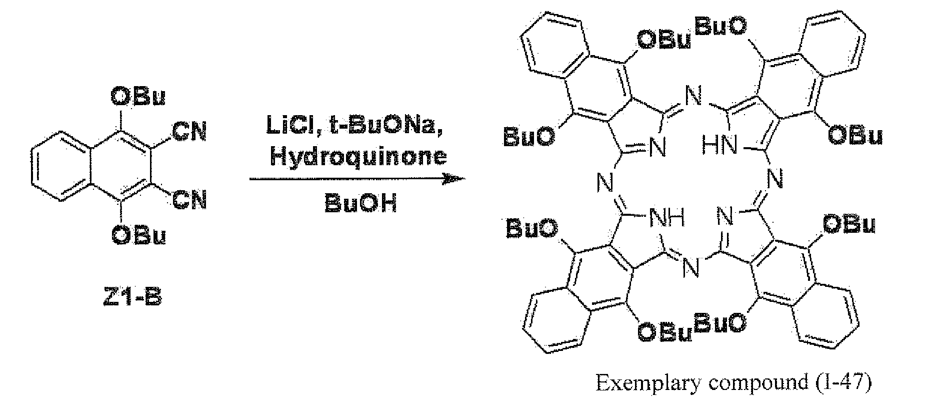

64.5 g of Exemplary compound (Z1-B), 50.9 g of lithium chloride, 48. 1 g of sodium t-butoxide, and 300 mL of butanol were added to a 2 L three neck flask, and heating was performed for 3 hours at 90°C. Subsequently, 3.5 g of hydroquinone was added thereto and heating was performed at the same temperature for 1 hour. Then, after the mixture was cooled to 50°C, 400 mL of water, 30 mL of acetic acid, and 1L of chloroform were added thereto to extract an organic layer. Subsequently, rinsing was carried out two times using 400 mL of water, and the organic layer thus obtained was dried with anhydrous magnesium sulfate and concentrated to 250 g with a rotary evaporator. To the condensate, 400 mL of hexane was added at an inside temperature of 50°C, and then cooling was performed to -10°C over 2 hours while stirring. Then, the resultant was left to stand at -10°C for 12 hours, and a produced crystal was filtered under a reduced pressure and washed with 300 mL of methanol, thereby obtaining 60.6 g of Exemplary compound (I-47) (yield of 94%). When the obtained compound was measured with an MS spectrum, a peak corresponding to the molecular weight was obtained.

### (Synthetic example 3)

### Synthesis of Exemplary compound (1-48) is shown.

Exemplary compound (I-48) was synthesized based on the following reaction scheme.

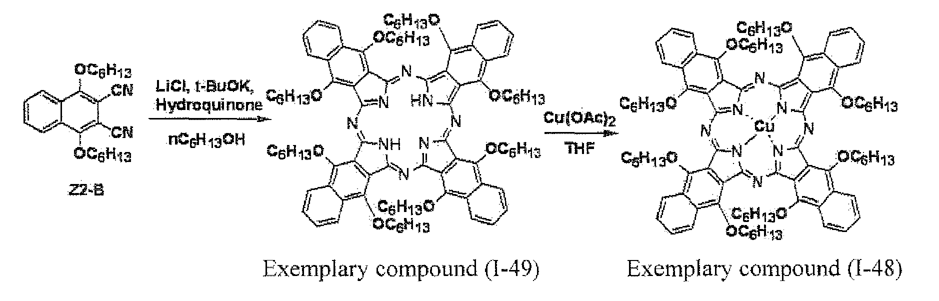

75.7 g of Exemplary compound (Z2-B) which has been synthesized in the same manner as Exemplary compound (Z1-B), 50.7 g of lithium chloride, 67.3 g of potassium t-butoxide, and 400 mL of hexanol were added to a 2 L three neck flask, and heated for 3 hours at 130°C. Subsequently, 27.5 g of hydroquinone was added thereto and heating was performed at the same temperature for 1 hour. Next, after the mixture was cooled to 50°C, 400 mL of water, 30 mL of acetic acid, and 1 L of toluene were added thereto to extract an organic layer. Subsequently, rinsing was carried out two times using 400 mL of water, and the organic layer thus obtained was dried with anhydrous magnesium sulfate and concentrated to 200 g with a rotary evaporator, To the condensate, 400 mL of hexane was added thereto at an inside temperature of 65°C, and then cooling was performed to 0°C over 2 hours while stirring. Then, the resultant was left to stand at 0°C for 2 hours, and a produced crystal was filtered under a reduced pressure and washed with 300 mL of methanol.

By performing the above operation, 67.5 g of Exemplary compound (1-49) was obtained (yield of 89%). When the obtained compound was measured with an MS spectrum, a peak corresponding to the molecular weight was obtained.

After 15.2 g of Exemplary compound (1-49) was dissolved in 1 L of THF, 2.0 g of copper acetate (2 equivalent amounts) was added thereto, and then heating was performed at 60°C for 8 hours. After the reaction solution was cooled, a crystal was precipitated by adding water. After the filtration, the crystal was rinsed with water, thereby obtaining 15.0 g of Exemplary compound (I-48) (yield of 95%).

### (Synthetic example 4)

### Synthesis of Exemplary compound (I-50) is shown.

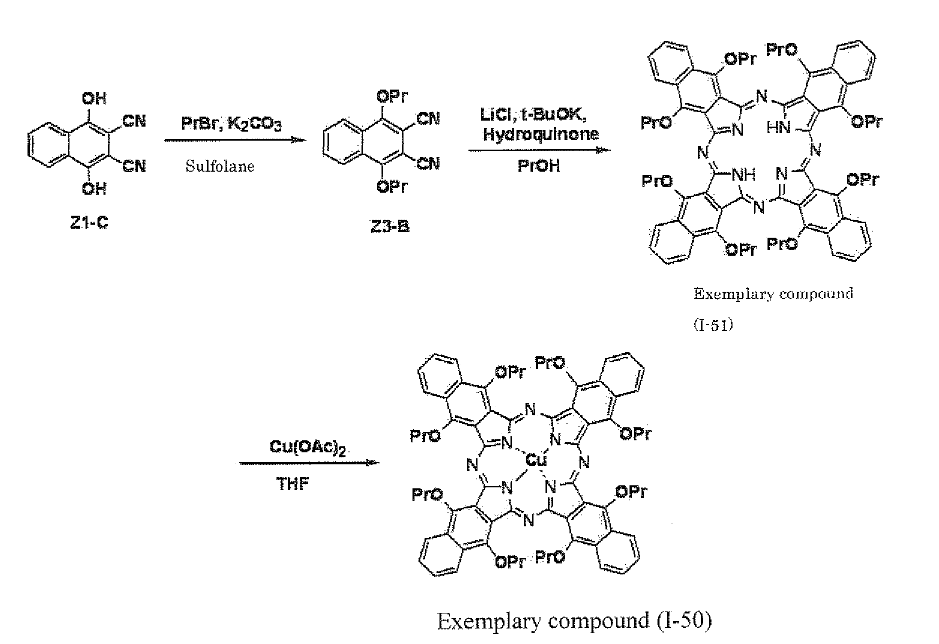

100.0 g of 2,3-dicyano-1,4-dihydronaphthoquinone (Z1-C), 128.6 g af propyl bromide, 131.4 g of potassium carbonate, and 350 mL of sulfolane were added to a 2 L three neck flash, and stirred for 4 hours at an inside temperature of 90°C. After cooling to an inside temperature of 40°C and adding 630 mL of water dropwise over 20 min, the mixture was cooled to 5°C over 1 hour. After the mixture was stirred at 5°C for 15 min, the obtained slurry was filtered and washed with 300 mL of methanol and 360 mL of water, thereby obtaining 130.9 g of Exemplary compound (Z3-B) (yield of 93%).

58.9 g of Exemplary compound (Z3-B), 50.8 g of lithium chloride, 56.1 g of potassium t-butoxide, and 400 mL of propanol were added to a 2 L three neck flask, and heated for 3 hours at 120°C. Subsequently. 16.5 g of hydroquinone was added thereto, and heating was performed at the same temperature for 1 hour. Next, cooling was performed to 50°C, 850 mL of water and 300 mL of acetic acid were added thereto, and the mixture was stirred for 20 min. The obtained slurry was filtered and washed with 160 mL of acetone, 160 mL of water, and 160 mL of methanol, thereby obtaining 54.8 g of Exemplary compound (I-51) (yield of 93%). When the obtained compound was measured with an MS spectrum, a peak corresponding to the molecular weight was obtained.

After 15.0 g of Exemplary compound (I-51) was dissolved in 150 mL of THF, 5.8 g of copper acetate (2.5 equivalent amounts) was added thereto, and then heating was performed at 60°C for 2 hours. After the reaction solution was cooled, a crystal was precipitated by adding water. After the filtration, the crystal was washed with 500 mL of acetone, 300 mL of water, and 300 mL of methanol, thereby obtaining 14.5 g of Exemplary compound (I-50) (yield of 92%).

### (Comparative synthetic example 1)

### Comparative synthetic example for Exemplary compound (I-47) is shown.

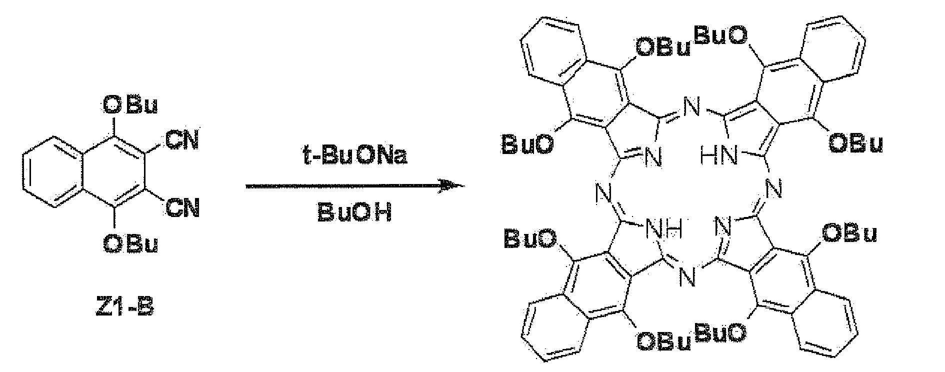

64.5 g of Exemplary compound (Z1-B), 48.1 g of sodium t-butoxide, and 300 mL of butanol were added to a 2 L three neck flask, and heated for 4 hours at 90°C. After the mixture was cooled to 50°C, 400 mL of water, 30 mL of acetic acid, and 1 L of chloroform were added thereto to extract an organic layer. Subsequently, rinsing was carried out two times using 400 mL of water, and the obtained organic layer was dried with anhydrous magnesium sulfate and concentrated to 250 g with a rotary evaporator. To the condensate, 400 mL of hexane was added at an inside temperature of 50°C, and then cooling was performed to -10°C over 2 hours while stirring. Then, the resultant was left to stand at -10°C for 12 hours, and a produced crystal was filtered under a reduced pressure and washed with 300 mL of methanol, thereby obtaining 16.1 g of Exemplary compound (1-47) (yield of 25%).

### (Comparative synthetic example 2)

Another comparative synthetic example for Exemplary compound (1-47) is shown.

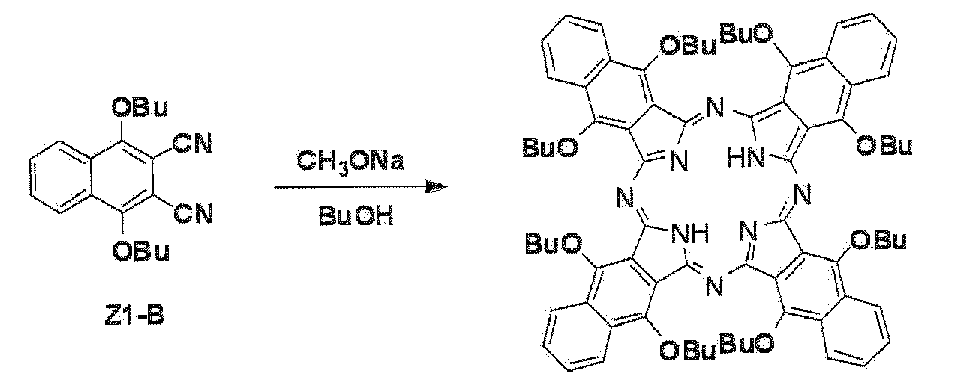

64.5 g of Exemplary compound (Z1-B), 27.0 g of sodium methoxide, and 300 mL of butanol were added to a 2 L three neck flask, and heated for 4 hours at 90°C. After the mixture was cooled to 50°C, 400 mL of water, 30 mL of acetic acid, and 1 L of chloroform were added thereto to extract an organic layer. Subsequently, rinsing was carried out two times using 400 mL of water, and the obtained organic layer was dried with anhydrous magnesium sulfate and concentrated to 250 g with a rotary evaporator. To the condensate, 400 mL of hexane was added at an inside temperature of 50°C, and then cooling was performed to -10°C over 2 hours while stirring. Then, the resultant was left to stand at -10°C for 12 hours, and a produced crystal was filtered under a reduced pressure and washed with 300 mL of methanol, thereby obtaining 11.6 g of a mixture of Exemplary compound (I-47) and Exemplary compound (I-47) in which a part of a butoxy group was converted into a methoxy group (yield of 19%).

### (Comparative synthetic example 3)

Another comparative synthetic example for Exemplary compound (1-47) is shown.

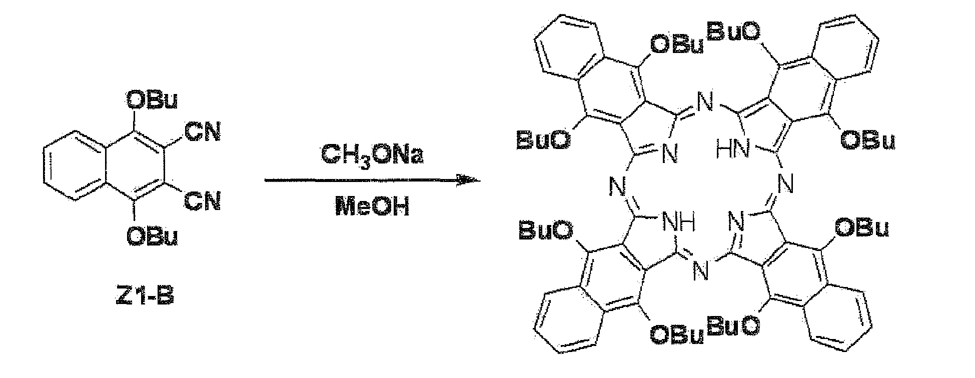

64.5 g of Exemplary compound (Z1-B), 27.0 g of sodium methoxide, and 300 mL of methanol were added to a 2 L three neck flask, and heated for 4 hours at 90°C. After the mixture was cooled to 50°C, 400 mL of water, 30 mL of acetic acid, and 1 L of chloroform were added thereto to extract an organic layer. Subsequently, rinsing was carried out two times using 400 mL of water, and the obtained organic layer was dried with anhydrous magnesium sulfate and concentrated to 250 g with a rotary evaporator. To the condensate, 400 mL af hexane was added at an inside temperature of 50°C, and then cooling was performed to -10°C over 2 hours while stirring. Then, the resultant was left to stand at - 10°C for 12 hours, and a produced crystal was filtered under a reduced pressure and washed with 300 mL of methanol, thereby obtaining 7.1 g of a mixture of Exemplary compound (I-47) and Exemplary compound (I-47) in which a portion of butoxy groups were converted into methoxy groups (yield of 1 1%).
This is another comparative synthetic example for Exemplary compound (I-47).

Among the synthetic examples described above, results of the cyclization reaction are summarized in Table 9.
According to the synthesis method of the invention, it is clear that high yield is obtained.

**[Table 9]**

| | Raw material (mole ratio) | Base (mole ratio) | Lithium halide (mole ratio) | Reaction solvent | Hydroquinone/ mole ratio | Yield |
|---|---|---|---|---|---|---|
| Synthetic example 1 | Z1-B (0.2) | t-BuONa (0.5) | LiCl (1.2) | n-BuOH | None | 72% |
| Synthetic example 2 | Z1-B (0.2) | t-BuONa (0.5) | LiCl (1.2) | n-BuOH | 0.1 | 94% |
| Synthetic example 3 | Z2-B (0.2) | t-BuOK (0.5) | LiCl (1.2) | n-C₆H₁₃OH | 0.25 | 89% |
| Synthetic example 4 | Z3-B (0.2) | t-BuOK (0.5) | LiCl (1.2) | n-PrOH | 0.15 | 93% |
| Comparative synthetic example 1 | Z1-B (0.2) | t-BuONa (0.5) | None | n-BuOH | None | 25% |
| Comparative synthetic example 2 | Z1_B (0.2) | CH₃ONa (0.5) | None | n-BuOH | None | 19% |
| Comparative synthetic example 3 | Z1-B (0.2) | CH₃ONa (0.5) | None | MeOH | None | 11% |

### INDUSTRIAL APPLICABILITY

The invention is suitable for various uses such as identification of originality (for example, types or authenticity of goods), signal detection, prevention of counterfeiting, welding of a polymer, and shielding of infrared rays, by using absorption in the infrared region, especially in the electromagnetic spectrum of a near infrared region of from 750 nm to 950 nm, and particularly from 800 nm to 900 nm, and for use in materials employed for such uses (for example, an ink, mineral oil, a heat ray-shielding material, and a plate for lithographic priding).

## Claims

1. A method of proving authenticity of goods or a support, comprising using a compound represented by the following Formula (I): 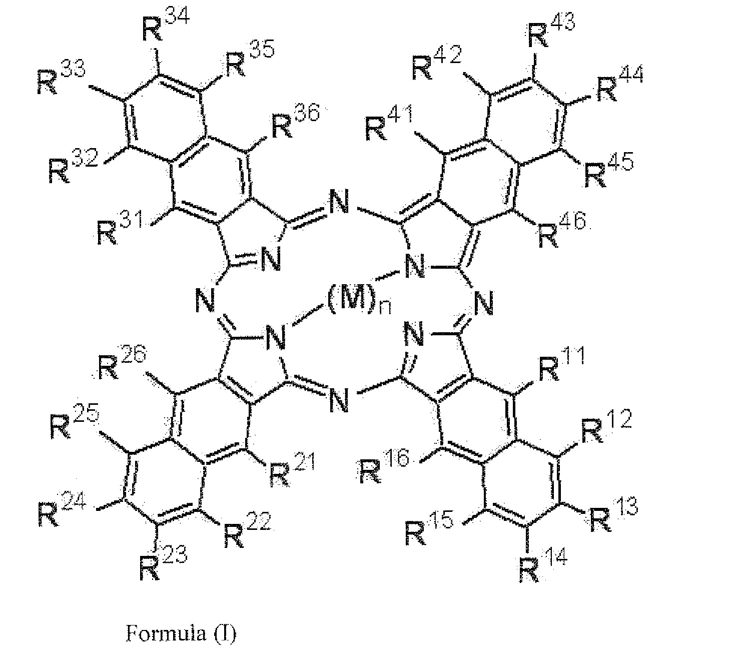 wherein, in Formula (1), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring; M represents a hydrogen atom, a metal ion, or a group containing a metal ion; and n represents 1 or 2.

2. The method of proving authenticity according to claim 1, wherein, in Formula (I), at least one of R¹¹, R¹⁶, R²¹, R²⁶, R³¹, R³⁶, R⁴¹, and R⁴⁶ represents an alkoxy group, an aryloxy group, an arylthio group, or a halogen atom.

3. The method of proving authenticity according to claim 1 or 2, wherein, in Formula (I), at least one of R¹¹, R¹⁶, R²¹, R²⁶, R³¹, R³⁶, R⁴¹, and R⁴⁶ represents an alkoxy group.

4. The method of proving authenticity according to any one of claims 1 to 3, comprising attaching, to a surface of the goods (or the support), an image using the compound represented by Formula (I), and detecting the image attached to the surface of the goods by irradiating with an infrared ray and scanning.

5. The method of proving authenticity according to any one of claims 1 to 3, comprising attaching, to the goods (or the support), a marking formed using the compound represented by Formula (I) and detecting or measuring a specific absorption by irradiating the marking with an infrared ray.

6. The method of proving authenticity according to claim 5, wherein the goods are mineral oil.

7. A signal conversion method comprising converting a thermal signal into an enhanced thermal signal or converting a light signal into a thermal signal, by using a compound represented by the following Formula (I): 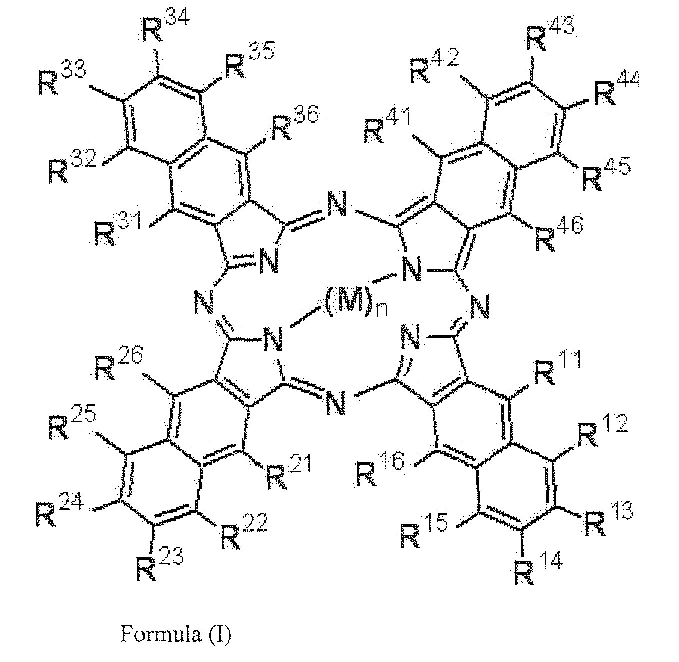 wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring; M represents a hydrogen atom, a metal ion, or a group containing a metal ion; and n represents 1 or 2.

8. The signal conversion method according to claim 7, wherein, in Formula (I), at least one of R¹¹, R¹⁶, R²¹, R²⁶, R³¹, R³⁶, R⁴¹, and R⁴⁶ represents an alkoxy group, an aryloxy group, an arylthio group, or a halogen atom.

9. The signal conversion method according to claim 7 or 8, wherein, in Formula (I), at least one of R¹¹, R¹⁶,R²¹, R²⁶, R³¹, R³⁶, R⁴¹, and R⁴⁶ represents an alkoxy group.

10. A method of welding a polymer using the signal conversion method according to any one of claims 7 to 9.

11. A method of producing a lithographic printing plate using the signal conversion method according to any one of claims 7 to 9, comprising irradiating a heat sensitive layer or photosensitive layer containing the compound represented by Formula (I) with an infrared laser in accordance with pattern information.

12. An ink for printing, comprising a compound represented by the following Formula (I): 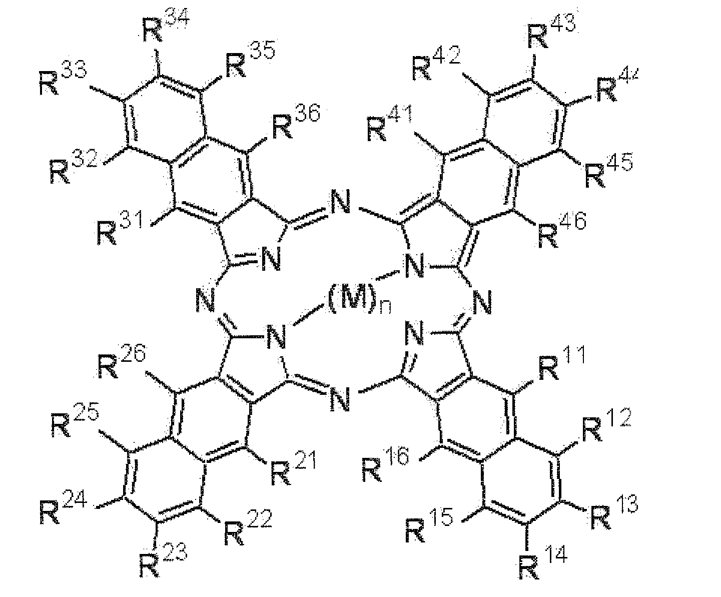 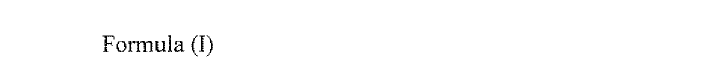 wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶ may be bonded to each other to form a ring; M represents a hydrogen atom, a metal ion, or a group containing a metal ion; and n represents 1 or 2.

13. The ink for printing according to claim 12, wherein, in Formula (I), at least one of R¹¹, R¹⁶, R²¹, R²⁶, R³¹, R³⁶, R⁴¹, and R⁴⁶ represents an alkoxy group, an aryloxy group, an arylthio group, or a halogen atoms.

14. The ink for printing according to claim 12 or 13, wherein, in Formula (I), at least one of R¹¹ , R¹⁶ , R²¹, R²⁶, R³¹, R³⁶, R⁴¹ and R⁴⁶ represents an alkoxy group.

15. The ink for printing according to any one of claims 12 to 14, further comprising a colorant.

16. The ink for printing according to any one of claims 12 to 15, further comprising an alkoxylated or polyalkoxylated acrylate monomer and a photoinitiator.

17. A toner comprising a compound represented by the following Formula (I): 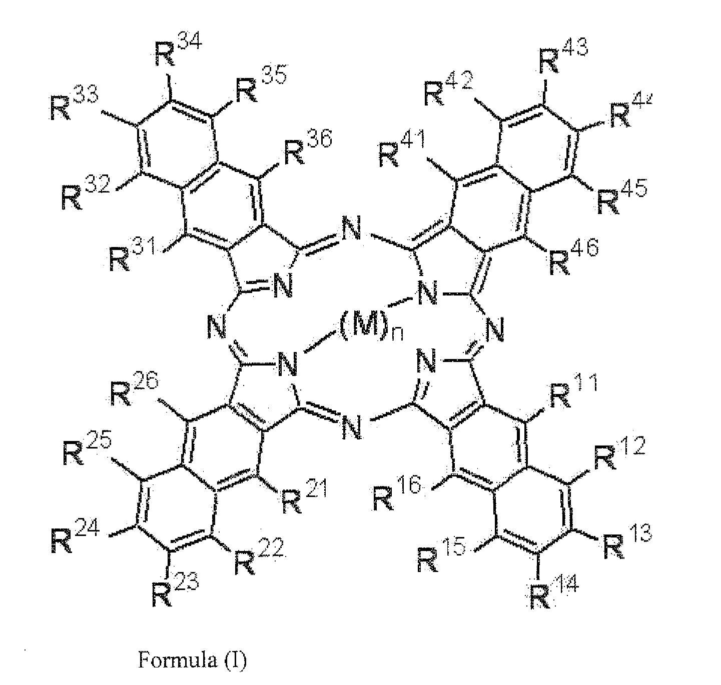 wherein, in Formula (I), each of R¹¹ to R⁴⁶ independently represents a hydrogen atom or a substituent group, wherein when a benzene ring is substituted with any of R¹¹ to R⁴⁶, any groups adjacent to each other among R¹¹ to R⁴⁶may be bonded to each other to form a ring; M represents a hydrogen atom, a metal ion, or a group containing a metal ion; and n represents 1 or 2.

18. The toner according to claim 17, wherein, in Formula (I), at least one of R¹¹, R¹⁶, R²¹, R²⁶, R³¹, R³⁶, R⁴¹, and R⁴⁶ represents an alkoxy group, an aryloxy group, an arylthio group, or a halogen atom.

19. The toner according to claims 17 or 18, wherein, in Formula (I), at least one of R¹¹, R¹⁶, R²¹, R²⁶, R³¹, R³⁶, R⁴¹, and R⁴⁶ represents an alkoxy group.

20. The toner according to any one of claims 17 to 19, further comprising a colorant.

21. The toner according to any one of claims 17 to 20, further comprising an alkoxylated or polyalkoxylated acrylate monomer and a photoinitiator.

22. A heat ray-shielding material comprising a compound represented by the following Formula (I): 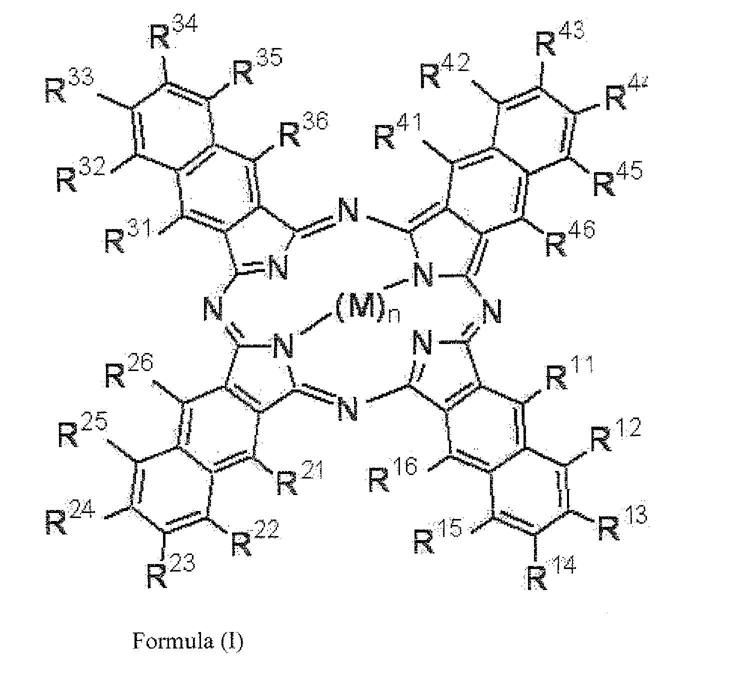 wherein, in Formula (I), each of R¹¹ to R⁴⁶ independently represents a hydrogen atom or a substituent group, wherein when a benzene ring is substituted with any of R¹¹ to R⁴⁶ , any groups adjacent to each other among R¹¹ to R⁴⁶ may be bonded to each other to form a ring; M represents a hydrogen atom, a metal ion, or a group containing a metal ion; and n represents 1 or 2.

23. The heat ray-shielding materials according to claim 22, wherein, in Formula (I), at least one of R¹¹, R¹⁶, R²¹, R²⁶, R³¹ R³⁶, R⁴¹, and R⁴⁶ represents an alkoxy group, an aryloxy group, an arylthio group, or a halogen atom.

24. The heat ray-shielding material according to claim 22 or 23, wherein, in Formula (I), at least one of R¹¹, R¹⁶, R²¹, R²⁶, R³¹, R³⁶, R⁴¹, and R⁴⁶ represents an alkoxy group.

25. A method of producing a compound represented by Formula (II) comprising using t-BuOM³ and lithium halide in a solvent represented by one of Formulae R¹OH to R⁸OH or a mixture solvent of two or more thereof: 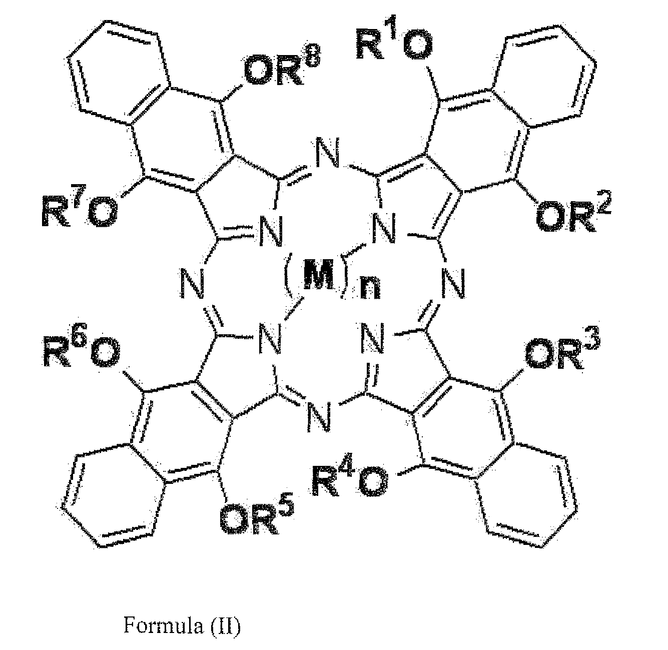 wherein, substituent groups R¹ to R⁸ each independently represent an alkyl group or an aryl group; M³ represents an alkali metal atom; M represents a hydrogen atom, a metal ion, or a group containing a metal ion; and n represents 1 or 2.

26. The method of producing a compound represented by Formula (II) according to claim 25 comprising using hydroquinone.
